# EUROPEAN PATENT SPECIFICATION

(11) **EP 1 447 447 B1**
(45) Date of publication and mention of the grant of the patent: **11.11.2009**
(21) Application number: 02777791.1
(22) Date of filing: 26.09.2002
(51) Int. Cl.: C12N 15/54, C12P 21/02, C12N 9/10, C12R 1/01

(54) **PROCESS FOR PRODUCING SIALIC ACID-CONTAINING COMPLEX SUGAR**
VERFAHREN ZUR HERSTELLUNG SIALSÄURE ENTHALTENDEM KOMPLEXEM ZUCKER
PROCEDE DE PRODUCTION DE SUCRE COMPLEXE CONTENANT DE L'ACIDE SIALIQUE

(30) Priority: 26.09.2001 JP 2001292796
(43) Date of publication of application: 18.08.2004
(73) Proprietor: Kyowa Hakko Bio Co., Ltd., Tokyo, 100-8185 (JP)
(72) Inventor: ENDO, Tetsuo, Stanford, CA 94305-6090 (US); KOIZUMI, Satoshi, Tokyo Research Laboratories, Machida-shi, Tokyo 194-8533 (JP)
(74) Representative: Vossius & Partner
(86) International application number: PCT/JP2002/009907
(87) International publication number: WO 2003/027297

(56) References cited:
- EP-A2- 0 736 602
- WO-A-00/46379
- JP-A- 11 253 163
- JP-A- 2000 333 682
- US-A- 6 096 529
- DATABASE UNIPROT/SWISS-PROT 1 June 2001 (2001-06-01), MAY, B.J. ET AL.: "Hypothetical protein PM0188" XP002320253 retrieved from EBI Database accession no. Q9CP67
- DATABASE UNIPROT/SWISSPROT 1 June 2001 (2001-06-01), MAY, B.J. ET AL.: "Hypothetical protein PM1174" XP002320254 retrieved from EBI Database accession no. Q9CLP3
- DATABASE EMBL 10 February 2001 (2001-02-10), MAY,B.KJ. ET AL.: "Pasteurella multocida subsp. multocida str. Pm70" XP002320255 retrieved from EPBI Database accession no. AE006053
- DATABASE EMBL 10 February 2001 (2001-02-10), MAY,B.J. ET AL.: "Pasteurella multocida subsp. multocida str. Pm70 section 53 of 204 of the complete genome" XP002320256 retrieved from EBI Database accession no. AE006086
- DATABASE EMBL 10 February 2001 (2001-02-10), MAY, B.J. ET AL.: "Pasteurella multocida subsp. multocida str. Pm70 section 124 of 204 of the complete genome" XP002320257 retrieved from EBI Database accession no. AE006157

## Description

### TECHNICAL FIELD

The present invention relates to a process for producing a protein having α2,3/α2,8-sialyltransferase activity using a transformant comprising a DNA encoding a protein having α2,3/α2,8-sialyltransferase activity and a process for producing a sialic acid-containing complex carbohydrate.

### BACKGROUND ART

As genes encoding α2,3-sialyltransferase genes derived from animals *[*J. Biol. Chem., 267, 21011 (1992), J. Biol. Chem., 268, 22782 (1993), Eur. J. Biochem., 216, 377 (1993), Biochem. Biophys. Res. Commun., 194, 375 (1993), J. Biol. Chem., 269, 1394 (1994), J. Biol. Chem., 269, 10028 (1994), Glycobiology, 5, 319 (1995)] and the like have been obtained. Although there is an example in which the gene encoding the α2,3-sialyltransferase derived from an animal was expressed in bacteria such as *Escherichia coli* and a protein having activity was obtained (Japanese Published Unexamined Patent Application No. 2531631/99), its activity was very weak. Also, as genes encoding α2,8-sialyltransferase genes derived from animals [Proc. Natl. Acad. Sci. USA, 91, 7952 (1994), Proc. Natl. Acad. Sci. USA, 91, 10455 (1994), J. Biol. Chem., 269, 15950 (1994), J. BioL Chem., 271, 3684 (1996), J. Biol. Chem., 270, 14628 (1995)] and the like have been obtained. However, there is no example in which the gene encoding the α2,8-sialyltransferase derived from an animal was expressed in bacteria such as *Escherichia coli* and a protein having activity was obtained.

On the other hand, in the case of microorganisms, there is a report in which a gene encoding the α2,3-sialyltransferase was obtained from microorganisms belonging to the genus *Neisseria,* the genus *Campylobacter* and the genus *Haemophilus* and the α2,3-sialyltransferase was expressed in *Escherichia coli* using the gene [USP 6,096,529, WO97/47749, WO99/49051, WO00/46379, J. Biol. Chem., 271, 28271 (1996), J. Biol. Chem., 275, 3896 (2000), Mol. Microbiol., 39, 341 (2001)]. However, there is no report in which the gene is obtained from a microorganism belonging to the genus *Pasteurella.*

Furthermore, the α2,3-sialyltransferase derived from a microorganism belonging to the genus *Campylobacter* has also α2,8-sialyltransferase activity [WO00/46379, J. Biol. Chem., 275, 3896 (2000)] and a process for producing a sialic acid-containing carbohydrate comprising the use of said sialyltransferase in the presence of an acceptor carbohydrate is described [WO00/46379]. However, it has not been known that there is a protein having α2,8-sialyltransferase activity in a microorganism belonging to the genus *Pasteurella.*

The full nucleotide sequence of the genomic DNA in *Pasteurella multocida* PM70 was determined [Proc. Natl. Acad Sci., USA, 98, 3460 (2001)] and the genomic DNA (Database GenBank accession number AE006053) contains the 1236 nucleotide sequence of SEQ ID NO:2. The corresponding amino acid sequence (SEQ ID NO:1) is identical to the sequence of hypothetical protein PM0188 (Database Uniprot/Swiss-prot accession number Q9CP67).

### DISCLOSURE OF THE INVENTION

The present invention relates to the following items.
1. A process for producing a sialic acid-containing complex carbohydrate, which comprises: allowing a culture of a transformant capable of producing a protein having α2,3/α2,8-sialyltransferase activity derived from a microorganism or a treated product of the culture as an enzyme source, cytidine-5'-monophosphosialic acid and an acceptor complex carbohydrate to be present in an aqueous medium to produce and accumulate the sialic acid-containing complex carbohydrate in the aqueous medium, and recovering the sialic acid-containing complex carbohydrate from the aqueous medium,
   wherein the protein having α2,3/α2,8-sialyltransferase activity is a protein selected from the group consisting of:
   (a) a protein comprising the amino acid sequence represented by SEQ ID NO: 1;
   (b) a protein which consists of an amino acid sequence in which 1 to 20 amino acids are deleted, substituted or added in the amino acid sequence represented by SEQ ID NO: 1, and has α2,3/α2,8-sialyltransferase activity; and
   (c) a protein which has an identity of at least 80% with the amino acid sequence represented by SEQ ID NO: 1, and has α2,3/α2,8-siatyltransferase activity.
2. The process according to item 1, wherein the treated product of the culture is selected from the group consisting of a concentrated product of the culture, a dried product of the culture, cells obtained by centrifuging the culture, a dried product of the cells, a freeze-dried product of the cells, a surfactant-treated product of the cells, an ultrasonic-treated product of the cells, a mechanically disrupted product of the cells, a solvent-treated product of the cells, an enzyme-treated product of the cells, a protein fraction of the cells, an immobilized product of the cells and an enzyme preparation obtained by extracting from the cells.
3. The process according to item 1, wherein the acceptor complex carbohydrate is a complex carbohydrate comprising an oligosaccharide having galactose in its non-reducing terminal.
4. The process according to item 1, wherein the acceptor complex carbohydrate is a complex carbohydrate comprising an oligosaccharide having sialic acid in its non-reducing terminal.
5. The process according to item 3, wherein the oligosaccharide having galactose in its non-reducing terminal is an oligosaccharide selected from the group consisting of lactose, globotriose, *N*-acetyllactosamine, lacto-*N*-tetraose, lacto-*N*-neotetraose, Lewis^{a} and Lewis^{x}.
6. The process according to item 4, wherein the oligosaccharide having sialic acid in its non-reducing terminal is an oligosaccharide selected from the group consisting of NeuAcα2-3Galβ1-4Glc and NeuAcα2-3Galβ1-4GlcNAc.
7. The process according to item 1, wherein the microorganism is a microorganism belonging to the genus *Pasteurella.*
8. The process according to item 7, wherein the microorganism belonging to the genus *Pasteurella* is *Pasteurella multocida.*
9. The process according to item 1, wherein the transformant is a transformant which comprises a recombinant DNA comprising a DNA encoding said protein having α2,3/α2,8-sialyltransferase activity.
10. The process according to item 9, wherein the transformant is obtained by using a microorganism as a host cell.
11. The process according to item 10, wherein the microorganism is *Escherichia coli.*
12. The process according to item 9, wherein the DNA encoding the protein having α2,3/α2,8-sialyltransferase activity is
   (a) a DNA comprising the nucleotide sequence represented by SEQ ID NO: 2; or
   (b) a DNA which hybridizes with a DNA consisting of a complementary sequence of the nucleotide sequence represented by SEQ ID NO: 2 under stringent conditions, and encodes a protein having α2,3/α2,8-sialyltransferase activity.

A process for producing a protein having α2,3/α2,8-sialyltransferase activity using a transformant comprising a DNA encoding a protein having α2,3/α2,8-sialyltransferase activity and a process for producing a sialic acid-containing complex carbohydrate is disclosed in the context of the present invention.

In order to solve the above problems, the present inventors have conducted intensive studies and obtained pm0188 gene product having homology with a known α2.6-sialyltransferase and pm0508, gene product and pm1174 gene product having homology with a known α2,3-sialyltransferase by searching the sequence information of *Pasteurella multocida* PM70 in which the sequence of the genomic DNA was determined. It was found that all gene products have α2,3/α2,8-sialyltransferase activity by studying the activity of each of the gene products in detail, the DNA was obtained, and thus the present invention has been completed.

Specifically, the following (1) to (29) is disclosed in the context of the present invention:
(1) A process for producing a protein having α2,3/α2,8-sialyltransferase activity, which comprises: culturing a transformant capable of producing a protein having α2,3/α2,8-sialyltransferase activity derived from a microorganism belonging to the genus *Pasteurella* in a medium to produce and accumulate the protein having α2,3/α2,8-sialyltransferase activity in the culture, and recovering the protein from the culture.
(2) The process according to (1), wherein the microorganism belonging to the genus *Pasteurella* is *Pasteurella multocida.*
(3) The process according to (1), wherein the protein having α2,3/α2,8-sialyltransferase activity is a protein comprising the amino acid sequence represented by any one selected from SEQ ID NOs:1, 3 and 5.
(4) The process according to (1), wherein the protein having α2,3/α2,8-sialyltransferase activity is a protein which consists of an amino acid sequence in which at least one amino acid is deleted, substituted or added in the amino acid sequence represented by any one selected from SEQ ID NOs:1, 3 and 5, and has α2,3/α2,8-siatyitransferase activity.
(5) The process according to (1), wherein the protein having α2,3/α2,8-sialyltransferase activity is a protein which has an identity of at least 80% with the amino acid sequence represented by any one selected from SEQ ID NOs:1, 3 and 5, and has α2,3/α2,8-sialyltransferase activity.
(6) The process according to (1), wherein the transformant is a transformant which comprises a recombinant DNA comprising a DNA encoding a protein having α2,3/α2,8-sialyltransferase activity.
(7) The process according to (6), wherein the transformant is obtained by using a microorganism as a host cell.
(8) The process according to (7), wherein the microorganism is *Escherichia coli.*
(9) The process according to (6), wherein the DNA encoding the protein having α2,3/α2,8-sialyltransferase activity is a DNA described in one of the following (a) to (e):
   (a) a DNA encoding a protein comprising the amino acid sequence represented by any one selected from SEQ ID NOs:1, 3 and 5;
   (b) a DNA encoding a protein which consists of an amino acid sequence in which at least one amino acid is deleted, substituted or added in the amino acid sequence represented by any one selected from SEQ ID NOs:1, 3 and 5, and has α2,3/α2,8-sialyltransferase activity;
   (c) a DNA encoding a protein which has an identity of at least 80% with the amino acid sequence represented by any one selected from SEQ ID NOs:1, 3 and 5, and has α2,3/α2,8-sialyltransferase activity;
   (d) a DNA comprising the nucleotide sequence represented by any one selected from SEQ ID NOs:2, 4 and 6; and
   (e) a DNA which hybridizes with a DNA consisting of a complementary sequence of the nucleotide sequence represented by any one selected from SEQ ID NOs:2, 4 and 6 under stringent conditions, and encodes a protein having α2,3/α2,8-sialyltransferase activity.
(10) A process for producing a sialic acid-containing complex carbohydrate, which comprises: allowing a culture of a transformant capable of producing a protein having α2,3/α2,8-sialyltransferase activity derived from a microorganism or a treated product of the culture as an enzyme source, cytidine-5'-monophosphosialic acid and an acceptor complex carbohydrate to be present in an aqueous medium to produce and accumulate the sialic acid-containing complex carbohydrate in the aqueous medium, and recovering the sialic acid-containing complex carbohydrate from the aqueous medium.
(11) The process according to (10), wherein the treated product of the culture is selected from the group consisting of a concentrated product of the culture, a dried product of the culture, cells obtained by centrifuging the culture, a dried product of the cells, a freeze-dried product of the cells, a surfactant-treated product of the cells, an ultrasonic-treated product of the cells, a mechanically disrupted product of the cells, a solvent-treated product of the cells, an enzyme-treated product of the cells, a protein fraction of the cells, an immobilized product of the cells and an enzyme preparation obtained by extracting from the cells.
(12) The process according to (10), wherein the acceptor complex carbohydrate is a complex carbohydrate comprising an oligosaccharide having galactose in its non-reducing terminal.
(13) The process according to (10), wherein the acceptor complex carbohydrate is a complex carbohydrate comprising an oligosaccharide having sialic acid in its non-reducing terminal.
(14) The process according to (12), wherein the oligosaccharide having galactose in its non-reducing terminal is an oligosaccharide selected from the group consisting of lactose, globotriose, N-acetyllactosamine, lacto-N-tetraose, lacto-N-neotetraose, Lewis^{a} and Lewis^{x}.
(15) The process according to (13), wherein the oligosaccharide having sialic acid in its non-reducing terminal is an oligosaccharide selected from the group consisting of NeuAcα2-3Galβ1-4Glc and NeuAcα2-3Galβ1-4GlcNAc.
(16) The process according to (10), wherein the microorganism is a microorganism belonging to the genus *Pasteurella.*
(17) The process according to (16), wherein the microorganism belonging to the genus *Pasteurella* is *Pasteurella multocida.*
(18) The process according to (10), wherein the protein having α2,3/α2,8-sialyltransferase activity is a protein comprising the amino acid sequence represented by any one selected from SEQ ID NOs:1, 3 and 5.
(19) The process according to (10), wherein the protein having α2,3/α2,8-sialyltransferase activity is a protein which consists of an amino acid sequence in which at least one amino acid is deleted, substituted or added in the amino acid sequence represented by any one selected from SEQ ID NOs:1, 3 and 5, and has α2,3/α2,8-sialyltransferase activity.
(20) The process according to (10), wherein the protein having α2,3/α2,8-sialyltransferase activity is a protein which has an identity of at least 80% with the amino acid sequence represented by any one selected from SEQ ID NOs:1, 3 and 5, and has α2,3/α2,8-sialyltransferase activity.
(21) The process according to (10), wherein the transformant is a transformant which comprises a recombinant DNA comprising a DNA encoding a protein having α2,3/α2,8-sialyltransferase activity.
(22) The process according to (21), wherein the transformant is obtained by using a microorganism as a host cell.
(23) The process according to (22), wherein the microorganism is *Escherichia coli.*
(24) The process according to (21), wherein the DNA encoding the protein having α2,3/α2,8-sialyltransferase activity is a DNA represented by one of the following (a) to (e):
   (a) a DNA encoding a protein comprising the amino acid sequence represented by any one selected from SEQ ID NOs:1, 3 and 5;
   (b) a DNA encoding a protein which consists of an amino acid sequence in which at least one amino acid is deleted, substituted or added in the amino acid sequence represented by any one selected from SEQ ID NOs:1, 3 and 5, and has α2,3/α2,8-sialyltransferase activity;
   (c) a DNA encoding a protein which has an identity of at least 80% with the amino acid sequence represented by any one selected from SEQ ID NOs:1, 3 and 5, and has α2,3/α2,8-sialyltransferase activity;
   (d) a DNA comprising the nucleotide sequence represented by any one selected from SEQ ID NOs:2,4 and 6; and
   (e) a DNA which hybridizes with a DNA consisting of a complementary sequence of the nucleotide sequence represented by any one selected from SEQ ID NOs:2, 4 and 6 under stringent conditions, and encodes a protein having α,3/α2,8-sialyltransferase activity.
(25) A protein having α2,3/α2,8-sialyltransferase activity, which comprises the amino acid sequence represented by SEQ ID NO:1.
(26) A protein having α2,3/α2,8-sialyltransferase activity, which comprises the amino acid sequence represented by SEQ ID NO:3.
(27) A protein having α2,3/α2,8-sialyltransferase activity, which comprises the amino acid sequence represented by SEQ ID NO:5.
(28) A protein which consists of an amino acid sequence in which at least one amino acid is deleted, substituted or added in the amino acid sequence represented by any one selected from SEQ ID NOs:1, 3 and 5, and has α2,3/α2,8-sialyltransferase activity.
(29) A protein which has an identity of at least 80% with the amino acid sequence represented by any one selected from SEQ ID NOs:1, 3 and 5, and has α2,3/α2,8-sialyltransferase activity.

The protein having α2,3/α2,8-sialyltransferase activity as disclosed in the context of the present invention includes the protein derived from a microorganism belonging to the genus *Pasteurella,* preferably the protein derived from *Pasteurella multocida.* The origin of the protein having α2,3/α2,8-sialyltransferase activity used in the process for producing a complex carbohydrate as disclosed in the context of the present invention is not particularly limited, so long as it is derived from a microorganism, and is preferably a protein derived from a microorganisrn belonging to the genus *Pasteurella,* and more preferably a protein derived from *Pasteurella multocida.* Specifically, the protein as disclosed in the context of the present invention is a protein comprising the amino acid sequence represented by any one selected from SEQ ID NOs:1, 3 and 5; a protein which consists of an amino acid sequence in which at least one amino acid is deleted, substituted or added in the amino acid sequence represented by any one selected from SEQ ID NOs:1, 3 and 5, and has α2,3/α,8-sialyltransferase activity; and a protein which has an identity of at least 80% with the amino acid sequence represented by any one selected from SEQ ID NOs:1, 3 and 5, and has α2,3/α2,8-sialyltransferase activity.

The protein which consists of an amino acid sequence in which at least one amino acid is deleted, substituted or added, and has α2,3/α2,8-sialyltransferase activity can be obtained, for example, by introducing mutation(s) to a DNA encoding a protein comprising the amino acid sequence represented by any one selected from SEQ ID NOs:1, 3 and 5 according to a method for introducing site-directed mutagenesis described in Molecular Cloning, A Laboratory Manual, Second Edition, Cold Spring Harbor Laboratory Press (1989) (hereinafter referred to as *"Molecular Cloning,* Second Edition"); Current Protocols in Molecular Biology, John Wiley & Sons (1987-1997) (hereinafter referred to as *"Current Protocols in Molecular Biology");* Nucleic Acids. Research, 10, 6487 (1982); Proc. Natl. Acad Sci. USA, 79, 6409 (1982); Gene, 34, 315 (1985); Nucleic Acids. Research, 13, 4431 (1985); Proc. Natl. Acad. Sci. USA, 82, 488 (1985) and the like.

The number of the amino acids which are deleted, substituted or added is not particularly limited; however, it is such a number that deletion, substitution or addition can be carried out by a known method such as method for introducing site-directed mutagenesis. The number is 1 to 20, more preferably 1 to 10, and most preferably 1 to 5.

The deletion, substitution or addition of at least one amino acid residue in the amino acid sequence represented by any one selected from SEQ ID NOs:1, 3 and 5 means that one or at least two amino acids are deleted, substituted or added at any position in the same sequence. The deletion, substitution or addition can be carried out in the same amino acid sequence simultaneously. Also, the amino acid residue substituted, inserted or added can be natural or non-natural. The natural amino acid residue includes L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, L-cysteine, and the like.

Herein, examples of amino acid residues which are substituted with each other are shown below. Amino acid residues in the same group can readily be substituted with each other.

### Group A:

leucine, isoleucine, norleucine, valine, norvaline, alanine, 2-aminobutanoic acid, methionine, *O*-methylserine, t-butylglycine, t-butylalanine, cyclohexylalanine;

### Group B:

aspartic acid, glutamic acid, isoaspartic acid, isoglutamic acid, 2-aminoadipic acid, 2-aminosuberic acid;

### Group C:

asparagine, glutamine;

### Group D:

lysine, arginine, ornithine, 2,4-diaminobutanoic acid, 2,3-diaminopropionic acid;

### Group E:

proline, 3-hydroxyproline, 4-hydroxyproline;

### Group F:

serine, threonine, homoserine;

### Group G:

phenylalanine, tyrosine.

Also, in order that the protein which consists of an amino acid sequence in which at least one amino acid is deleted, substituted or added has α2,3/α2,8-sialyltransferase activity, the protein has a homology of preferably at least 60% or more, more preferably 80% or more, and most preferably 95% or more, with the amino acid sequence represented by any one selected from SEQ ID NOs:1, 3 and 5.

The identity of an amino acid sequence or a nucleotide sequence can be determined by using the algorithm BLAST by Karlin and Altschul [Proc. Natl. Acad Sci. USA, 90,5873 (1993)] or FASTA [Methods Enzymol., 183, 63 (1990)]. The programs called BLASTN and BLASTX have been developed based on the above algorithm BLAST [J. Mol. Biol., 215, 403 (1990)]. In the case of analyzing a nucleotide sequence by BLASTN based on BLAST, for example, the parameter can be set to score = 100, wordlength = 12. Also, in the case of analyzing an amino acid sequence by BLASTX based on BLAST, for example, the parameter can be set to score = 50, wordlength = 3. When BLAST and Gapped BLAST programs are used, a default parameter of each program can be used. The specific analysis methods of using the above programs are known (http://www.ncbi.ntm.nih.gov.).

The transformant used in the production of the protein having α2,3/α2,8-sialyltransferase activity as disclosed in the context of the present invention is not particularly limited, so long as it is a transformant capable of producing a protein having α2,3/α2,8-sialyltransferase activity derived from a microorganism belonging to the genus *Pasteurella.* The transformant is preferably a transformant comprising a DNA encoding the protein derived from a microorganism belonging to the genus *Pasteurella,* and more preferably a transformant comprising a DNA encoding the protein derived from *Pasteurella multocida.* Examples include:
(1) a DNA encoding a protein comprising the amino acid sequence represented by SEQ ID NO:1;
(2) a DNA encoding a protein comprising the amino acid sequence represented by SEQ ID NO:3;
(3) a DNA encoding a protein comprising the amino acid sequence represented by SEQ ID NO:5;
(4) a DNA comprising the nucleotide sequence represented by SEQ ID NO:2;
(5) a DNA comprising the nucleotide sequence represented by SEQ ID NO:4;
(6) a DNA comprising the nucleotide sequence represented by SEQ ID NO:6;
(7) a DNA encoding a protein which consists of an amino acid sequence in which at least one amino acid is deleted, substituted or added in the amino acid sequence represented by any one selected from SEQ ID NOs:1, 3 and 5, and has α2,3/α2,8-sialyltransferase activity;
(8) a DNA encoding a protein which has the identity of at least 80% with the amino acid sequence represented by any one selected from SEQ ID NOs:1, 3 and 5, and has α2,3/α2,8-sialyltransferase activity; and
(9) a DNA which hybridizes with a DNA consisting of a complementary sequence of the nucleotide sequence represented by any one selected from SEQ ID NOs:2, 4 and 6 under stringent conditions, and encodes a protein having α2,3/α2,8-sialyltransferase activity.

The DNA which is hybridizable under stringent conditions is a DNA obtained by colony hybridization, plaque hybridization, Southern hybridization or the like using, as a probe, a part or a full length of a DNA consisting of a complementary sequence of the nucleotide sequence represented by any one selected from SEQ ID NOs:2, 4 and 6. Specifically, the DNA includes a DNA which can be identified by carrying out hybridization at 65°C in the presence of 0.7-1.0 mol/l NaCl using a filter on which a DNA prepared from colonies or plaques is immobilized, and then washing the filter with 0.1× to 2x SSC solution (the composition of 1× SSC solution contains 150 mmol/l sodium chloride and 15 mmol/l sodium citrate) at 65°C. The hybridization can be carried out in accordance with a known method described in, for example, *Molecular Cloning,* Second Edition; *Current Protocols in Molecular Biology;* DNA Cloning 1: Core Techniques, A Practical Approach, Second Edition, Oxford University (1995) or the like.

Specifically, the DNA which is hybridizable includes a DNA having a homology of at least 60% or more, preferably 80% or more, and more preferably 95% or more, with the nucleotide sequence represented by any one selected from SEQ ID NOs:2, 4 and 6 when calculated based on the above parameters using above BLAST, FASTA or the like.

### [1] Preparation of a DNA used in the process for producing the protein having α2,3/α2,8-sialyltransferase activity as disclosed in the context of the present invention

### (1) Selection of a DNA encoding the protein having α2,3/α2,8-sialyltransferase activity using database

The full nucleotide sequence of the genomic DNA in *Pasteurella multocida* PM70 was determined [Proc. Natl. Acad. Sci., USA, 98, 3460 (2001)], and the DNA encoding the protein having α2,3/α2,8-sialyltransferase activity can be selected by carrying out gene search, homology search and the like by using a known sialyltransferase gene based on the genomic DNA sequence database [http://www.cbc.umn.edu/ResearchProjects/Pm/pmhome.html, http://www.ncbi.nlm.nih.gov /BLAST/].

### (2) Preparation of a DNA encoding the protein having α2,3/α2,8-sialyltransferase activity

The DNA encoding the protein having α2,3/α2,8-sialyltransferase activity can be prepared from a microorganism belonging to the genus *Pasteurella.* The microorganism belonging to the genus *Pasteurella* includes *Pasteurella multocida,* and specifically *Pasteurella multocida* PM70 (available from Minnesota University) and the like.

The microorganism belonging to *Pasteurella multocida* can be cultured by a known method [for example, FEMS Microbiol. Lett., 166, 289 (1998)].

After the culturing, a chromosomal DNA of the microorganism can be isolated and purified by a known method (for example, method described in *Current Protocols in Molecular Biology*)*.*

A DNA fragment containing the DNA used in the production process for production as disclosed in the context of the present invention can be obtained by preparing a primer based on the nucleotide sequence of the genomic DNA selected in the item (1) and then carrying out PCR [PCR Protocols, Academic Press (1990)] using the genomic DNA as a template.

Furthermore, the DNA of interest can be obtained according to a hybridization method by using the synthetic DNA designed based on the nucleotide sequence of the genomic DNA as a probe.

The nucleotide sequence of the DNA can be determined by inserting the obtained DNA as it is or after digestion with an appropriate restriction enzyme, into a vector according to the usual method, and carrying out analysis by the generally used nucleotide sequence analysis method such as the dideoxy method [Proc. Natl. Acad. Sci. USA, 74, 5463 (1977)] or a method comprising the use of an apparatus for nucleotide sequence analysis such as 373A-DNA Sequencer (manufactured by Perkin-Elmer).

Based on the nucleotide sequence thus determined, the DNA of interest can also be prepared by chemical synthesis using, for example, DNA Synthesizer 8905 manufactured by Perseptive Biosystems or the like.

The thus obtained DNA includes a DNA comprising the sequence represented by SEQ ID NO:2, 4 or 6.

The vector into which the DNA of the present invention is ligated includes pBluescript II KS(+) (manufactured by Stratagene), pDIRECT [Nucleic Acids Res., 18, 6069 (1990)], pCR-ScriptAmp SK(+) (manufactured by Stratagene), pT7Blue manufactured by Novagen), pCR II (manufactured by Invitrogen), pCR-TRAP (manufactured by Genehunter) and the like.

Recombinant DNAs pPM0188SK, pPM0508SK and pPM1174SK which comprise the DNA comprising the nucleotide sequence represented by SEQ ID NO:2, 4 or 6, respectively, have been deposited on September 13, 2001, as FERM BP-7730, FERM BP-7731 and FERM BP-7733, respectively, in International Patent Organism Depositary, National Institute of Advanced Industrial Science and Technology (Central 6, 1-1, Higashi 1-Chome Tsukuba, Ibaraki, 305-8566, Japan).

The microorganism containing the recombinant DNA which comprises the DNA comprising the sequence represented by SEQ ID NO:2, 4 or 6 as disclosed in the context of the present invention is *Escherichia coli* and the like.

*Escherichia coli* includes *Escherichia coli* XLI-Blue, *Escherichia coli* XL2-Blue, *Escherichia coli* DH1, *Escherichia coli* MC1000, *Escherichia coli* KY3276, *Escherichia coli* W 1485, *Escherichia coli* JM-109, *Escherichia coli* HB 101, *Escherichia coli* No.49, *Escherichia coli* W3110, *Escherichia coli* NY49, *Escherichia coli* MP347, *Escherichia coli* NM522, *Escherichia coli* ME8415 and the like.

Any method can be used in the introduction method of the recombinant DNA, so long as it is a method for introducing DNA into the host cell. Examples include the method using a calcium ion [Proc. Natl. Acad. Sci. USA, 69, 2110 (1972)], the protoplast method (Japanese Published Unexamined Patent Application No. 248394/88), electroporation [Nucleic Acid Res., 16, 6127 (1988)] and the like.

The transformant capable of producing α2,3/α2,8-sialyltransferase obtained by introducing the above recombinant DNA includes a transformant containing the above recombinant DNA, a transformant into which a DNA encoding α2,3/α.2,8-sialyltransferase has been integrated into a chromosomal DNA of the host cell by homologous recombination, and the like.

The transformant containing the recombinant DNA includes *Escherichia coli* NM522/pPT76. *Escherichia coli* NM522/pPT79 and *Escherichia coli* NM522/pPT78 which are *Escherichia coli* strains containing the recombinant DNA which comprises the DNA comprising the nucleotide sequence represented by SEQ ID NO:2, 4 or 6, respectively.

### [2] Preparation of the protein having αc2,3/α2,8-sialyltransferase activity

The protein having α2,3/α2,8-sialyltransferase activity can be produced by expressing the DNA obtained by the method of the above item [1] in a host cell, for example, as shown below, by using a method described in *Molecular Cloning,* Second Edition, *Current Protocols in Molecular Biology* or the like.

Based on the above DNA, a DNA fragment of an appropriate length containing a portion which encodes the protein can be prepared, if necessary. In addition, productivity of the protein can be improved by substituting a nucleotide in the nucleotide sequence of the protein-coding region so that it has the most suitable codons for the expression in the host.

A recombinant DNA is prepared by inserting the DNA downstream of the promoter of an appropriate expression vector.

A transformant capable of producing the protein used for the process as disclosed in the context of the present invention can be obtained by introducing the recombinant DNA into a host cell suitable for the expression vector.

Any bacteria, yeasts, animal cells, insect cells, plant cells and the like can be used as the host cell, so long as it can express the gene of interest.

The expression vectors include those which can replicate autonomously, in the above host cell or those which can be integrated into a chromosome and have a promoter at such a position that the DNA encoding the protein having α2,3/α2,8-sialyltransferase activity can be transcribed.

When a procaryote such as bacterium is used as the host cell, it is preferred that the recombinant DNA which comprises the DNA encoding the protein used in the process for production as disclosed in the context of the present invention can replicate autonomously in the procaryote, and that the recombinant vector contains a promoter, a ribosome binding sequence, the DNA of the present invention and a transcription termination sequence. The vector may further comprise a gene regulating the promoter.

The expression vector includes pHelix 1 (manufactured by Roche Diagnostics), pKK233-2 (manufactured by Amersham Pharmacia Biotech), pSE280 (manufactured by Invitrogen), pGEMEX-1 (manufactured by Promega), pQE-8 (manufactured by QIAGEN), pET-3 (manufactured by Novagen), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(+), pBluescript II KS(-) (manufactured by Stratagene), pTrS30 [prepared from *Escherichia coli* JM109/pTrS30 (FERM BP-5407)], pTrS32 [prepared from *Escherichia coli* JM109/pTrS32 (FERM BP-5408)], pPAC31 (WO98/12343), pUC19 *[*Gene, 33, 103 (1985)], pSTV28 (manufactured by Takara Shuzo), pUC118 (manufactured by Takara Shuzo); pPA1 (Japanese Published Unexamined Patent Application No. 233798/88) and the like.

Any promoter can be used, so long as it can function in the host cell. Examples include promoters derived from *Escherichia coli,* phage and the like, such as *trp* promoter (P*ₜᵣₚ*), *lac* promoter (P*_{tac}*), P_{L} promoter, P_{R} promoter and P_{SE} promoter, SPO1 promoter, SPO2 promoter, penP promoter and the like. Also, artificially designed and modified promoters, such as a promoter in which two P*ₜᵣₚ* are linked in tandem, *tac* promoter, *lac*T7 promoter and *letI* promoter, can be used.

It is preferred to use a plasmid in which the space between Shine-Dalgarno sequence, which is the ribosome binding sequence, and the initiation codon is adjusted to an appropriate distance (for example, 6 to 18 nucleotides).

The transcription termination sequence to express the protein having α2,3/α2,8-sialyltransferase activity is not essential for the recombinant DNA. However, it is preferred to place a transcription terminating sequence immediately downstream of the structural gene.

The procaryotes include microorganisms belonging to the genera *Escherichia, Serratia, Bacillus, Brevibacterium, Corynebacterium, Microbacterium, Pseudomonas* and the like. Examples include *Escherichia coli* XLI-Blue, *Escherichia coli* XL2-Blue, *Escherichia coli* DI-il, *Escherichia coli* NM522, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Serratia ficaria, Serratia fonticola, Serratia liquefaciens, Serratia marcescens, Bacillus subtilis, Bacillus amyloliquefaciens, Brevibacterium immariophilum* ATCC 14068, *Brevibacterium saccharolyticum* ATCC 14066, *Corynebacterium ammoniagenes, Corynebacterium glutamicum* ATCC 13032, *Corynebacterium glutamicum* ATCC 14067, *Corynebacterium glutamicum* ATCC 13869, *Corynebacterium acetoacidophilum* ATCC 13870, *Microbacterium ammoniaphilum* ATCC 15354, *Pseudomonas* sp. D-0110 and the like.

Introduction of the recombinant DNA can be carried out by any methods for introducing DNA into the above-described host cells; such as the method using a calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

When a yeast cell is used as the host cell, the expression vector includes YEp13 (ATCC 37115), YEp24 (ATCC 37051), YCp50 (ATCC 37419), pHS19, pHS15 and the like.

Any promoter can be used so long as it can function in yeast. Examples include PH05 promoter, PGK promoter, GAP promoter, ADN promoter, gal 1 promoter, gal 10 promoter, a heat shock polypeptide promoter, MFα1 promoter, CUP 1 promoter and the like.

The host cell includes yeast strains belonging to the genera *Saccharomyces, Schizosaccharomyces, Kluyveromyces, Trichosporon, Schwanniomyces, Pichia, Candida* and the like. Examples include *Saccharomyces cerevisiae, Schizosaccharomyces pombe, Kluyveromyces lactis, Trichosporon pullulans, Schwanniomyces alluvius, Pichia pastoris, Candida utilis* and the like.

Introduction of the recombinant DNA can be carried out by any of the methods for introducing DNA into yeast, such as electroporation [Methods. Enzymol., 194, 182 (1990)], the spheroplast method [Proc. Natl. Acad Sci. USA, 81, 4889 (1984)] and the lithium acetate method [J. Bacteriol., 153, 163 (1983)].

When an animal cell is used as the host, the expression vector includes pcDNAI (available from Funakoshi)and pcDM8 (available from Funakoshi), pAGE107 (Japanese Published Unexamined Patent Application No. 22979/91), pAS3-3 (Japanese Published Unexamined Patent Application No. 227075/90), pcDNAI/Amp (manufactured by Invitrogen), pREP4 (manufactured by Invitrogen), pAGE103 [J. Biochem., 101, 1307 (1987)], pAGE210, pAMo, pAMoA and the like.

Any promoter can be used, so long as it can function in an animal cell. Examples include a promoter of IE (immediate early) gene of cytomegalovirus (CMV), SV40 early promoter, a metallothionein promoter, a promoter of retrovirus, a heat shock promoter, SRα promoter and the like. Also, the enhancer of the IE gene of human CMV can be used together with the promoter.

The host cell includes mouse myeloma cell, rat myeloma cell, mouse hybridoma cell, human Namalwa cell, Namalwa KJM-1 cell, human fetal kidney cell, human leukemia cell, African grivet kidney cell, Chinese hamster ovary (CHO) cell, HST5637 (Japanese Published Unexamined Patent Application No. 299/88) and the like.

The mouse myeloma cell includes SP2/0, NS0 and the like. The rat myeloma cell includes YB2/0 and the like. The human fetal kidney cell includes HEK293 (ATCC: CRL-1573) and the like. The human leukemia cell includes BALL-1 and the like. The African grivet kidney cell includes COS-1, COS-7 and the like.

Introduction of the recombinant DNA into animal cells can be carried out by any of methods for introducing DNA into animal cells, such as electroporation [Cytotechnology, 3, 133 (1990)], the calcium phosphate method (Japanese Published Unexamined Patent Application No. 227075/90), the lipofection method [Proc. Natl. Acad. Sci. USA, 84, 7413 (1987)], and the method described in Virology, 52, 456 (1973).

When an insect cell is used as the host, the protein can be produced by a known method described in, for example, Baculovir-us Expression Vectors, A Laboratory Manual, W.H. Freeman and Company, New York (1992), Molecular Biology, A Laboratory Manual, Current Protocols in Molecular Biology, Bio/Technology, 6, 47 (1988) or the like.

Specifically, a recombinant gene transfer vector and baculovirus are co-transfected into an insect cell to obtain a recombinant virus in a supernatant of the culture of its insect cell, and then an insect cell is infected with the resulting recombinant virus to produce the protein.

The transfer vector used in the method includes pVL1392, pVL1393 and pBlueBacIII (all manufactured by Invitrogen), and the like.

The baculovirus includes *Autographa californica* nuclear polyhedrosis virus which infects insects of the family *Barathra* and the like.

The insect cell includes *Spodoptera frugiperda* ovary cell, *Trichoplusia ni* ovary cell, silkworm ovary-derived culturing cell and the like.

*Spodoptera frugiperda* ovary cell includes Sf9 and Sf21 (*Baculovirus Expression Vectors, A Laboratory Manual*) and the like. *Trichoplusia ni* ovary cell includes High 5 and BTI-TN-5B1-4 (manufactured by Invitrogen) and the like. The cell line derived from silkworm ovary cell includes *Bombyx mori* N4 and the like.

The method for co-transfecting the above transfer vector and the above baculovirus for the preparation of the recombinant virus includes the calcium phosphate method (Japanese Published Unexamined Patent Application No. 227075/90), the lipofection method [Proc. Natl. Acad. Sci. USA, 84, 7413 (1987)] and the like.

When a plant cell is used as the host cell, the expression vector includes Ti plasmid, a tobacco mosaic virus vector, and the like.

As the promoter, any promoter can be used, so long as it can function in a plant cell. Examples include 35S promoter of cauliflower mosaic virus (CaMV), rice actin 1 promoter and the like.

The host cell includes a plant cell and the like, such as tobacco, potato, tomato, carrot, soybean, rape, alfalfa, rice, wheat and barley.

Introduction of the recombinant vector is carried out by any of methods for introducing DNA into a plant cell, such as *Agrobacterium* method (Japanese Published Unexamined Patent Application No. 140885/84, Japanese Published Unexamined Patent Application No. 70080/85, WO 94/00977), electroporation (Japanese Published Unexamined Patent Application No. 251887/85) and the method using a particle gun (Japanese Patent Nos. 2606856 and 2517813).

The protein having α2,3/α2,8-sialyltransferase activity can be produced by culturing the transformant thus obtained in a medium to produce and accumulate the protein having α2,3/α2,8-sialyltransferase activity in the culture, and recovering it from the culture.

Culturing the transformant used in the process for producing the protein having α2,3/α2,8-sialyltransferase activity in a medium is carried out according to the conventional method as used in culturing, of the host.

As a medium for culturing the transformant obtained by using, as the host, prokaryote such as *Escherichia coli,* or eukaryote such as yeast, either a natural medium or a synthetic medium may be used, so long as it contains a carbon source, a nitrogen source, an inorganic salt and the like which can be assimilated by the organism and the transformant can be cultured efficiently.

Any carbon source can be used, so long as the organism can assimilate it and it includes carbohydrates, such as glucose, fructose, sucrose, molasses containing them, starch and starch hydrolysate; organic acids, such as acetic acid and propionic acid; alcohols, such as ethanol and propanol; and the like.

The nitrogen source includes ammonia, various ammonium salts of inorganic acids or organic acids, such as ammonium chloride, ammonium sulfate, ammonium acetate and ammonium phosphate; other nitrogen-containing compounds; peptone; meat extract; yeast extract; corn steep liquor; casein hydrolysate; soybean meal and soybean meal hydrolysate; various fermented cells and digested matter thereof; and the like.

The inorganic salt includes potassium dihydrogen phosphate, dipotassium hydrogen phosphate, magnesium phosphate, magnesium sulfate, sodium chloride, ferrous sulfate, manganese sulfate, copper sulfate, calcium carbonate and the like.

Culturing is usually carried out under aerobic conditions by shaving culture, submerged spinner culture under aeration or the like. The culturing temperature is preferably from 15 to 40°C, and the culturing time is generally from 5 hours to 7 days. The pH of the medium is preferably maintained at 3.0 to 9.0 during the culturing. The pH can be adjusted using inorganic or organic acid, an alkali solution, urea, calcium carbonate, ammonia or the like.

Also, antibiotics, such as ampicillin and tetracycline, can be added to the medium during culturing, if necessary.

When a microorganism transformed with an expression vector containing an inducible promoter is cultured, an inducer can be added to the medium, if necessary. For example, isopropyl-β-D-thiogalactopyranoside or the like can be added to the medium when a microorganism transformed with an expression vector containing *lac* promoter is cultured; or indoleacrylic acid or the like can be added thereto when a microorganism transformed with an expression vector containing *trp* promoter is cultured.

The medium for culturing a transformant obtained using an animal cell as the host includes generally used RPMI 1640 medium [The Journal of the American Medical Association, 199, 519 (1967)], Eagle's MEM medium *[*Science, 122, 501 (1952)], DMEM medium [Virology, 8, 396 (1959)], and 199 Medium [Proceeding of the Society for the Biological Medicine, 73, 1 (1950)], as well as media to which fetal calf serum or the like has been added to the above media and the like.

Culturing is generally carried out at pH 6 to 8 and at 25 to 40°C for 1 to 7 days in the presence of 5% CO₂ or the like.

Furthermore, if necessary, antibiotics such as kanamycin, penicillin and streptomycin, can be added to the medium during the culturing.

The medium for culturing a transformant obtained using an insect cell as the host includes generally used TNM-FH medium (manufactured by Pharmingen), Sf-900 II SFM (manufactured by Life Technologies), ExCell 400 and ExCell 405 (both manufactured by JRH Biosciences), Grace's Insect Medium [Nature, 195, 788 (1962)] and the like.

Culturing is generally carried out at pH 6 to 7 and at 25 to 30°C for 1 to 5 days or the like.

Furthermore, if necessary, antibiotics such as gentamicin can be added to the medium during the culturing.

A transformant obtained by using a plant cell as the host cell can be used as the cell or after differentiating to a plant cell or organ. The medium used in the culturing of the transformant includes Murashige and Skoog (MS) medium, White medium, media to which a plant hormone, such as auxin or cytokinine, has been added, and the like.

Culturing is carried out generally at a pH 5 to 9 and at 20 to 40°C for 3 to 60 days.

Also, antibiotics, such as kanamycin and hygromycin, can be added to the medium during the culturing, if necessary.

As described above, the protein having α2,3/α2,8-sialyltransferase activity can be produced by culturing a transformant derived from a microorganism, animal cell, insect cell or plant cell containing the recombinant DNA which comprises the DNA encoding the protein according to the general culturing method to produce and accumulate the protein, and recovering the protein from the culture.

In the production of the protein having α2,3/α2,8-sialyltransferase activity, the protein can be produced so as to have a structure as it is or can be produced as a secretory protein having a signal sequence or a fusion protein according to the method described in *Molecular Cloning,* Second Edition and the like.

The protein to be fused includes β-galactosidase, protein A, IgG binding region of protein A, chloramphenicol acetylransferase, poly(Arg), poly(Glu), protein G, maltose binding protein, glutathione S-transferase, polyhistidine chain (His-tag), S peptide, DNA binding protein domain, Tac antigen, thioredoxin, green fluorescent protein, FLAG peptide, epitope of, any antibody, and the like [Akio Yamakawa, Experimental Medicine (Jikken Igaku), 13, 469-474 (1995)].

Furthermore, the process for producing the protein having α2,3/α2,8-siaiyltransferase activity includes a process for production in which the protein is produced on an outer membrane of the host cell, and the process for production can be selected by changing the host cell used or the structure of the protein produced.

When the protein having α2,3/α2,8-sialyltransferase activity is produced in a host cell or on an outer membrane of the host cell, the produced protein can be actively secreted extracellularly according to, for example, the method of Paulson *et al.* [J. Biol. Chem., 264, 17619 (1989)], the method of Lowe *et al.* [Proc. Natl. Acad. Sci. USA, 86, 8227 (1989); Genes Develop., 4, 1288 (1990), or the methods described in Japanese Published Unexamined Patent Application No. 336963/93, WO94/23021, and the like.

Specifically, the protein having α2,3/α,8-sialyltriransferase activity can be actively secreted extracellularly by producing it in the form that a signal peptide has been added to the N-terminus of a protein containing an active site of the protein having α2,3/α2,8-sialyltransferase activity according to the recombinant DNA technique.

Furthermore, the protein production can be increased utilizing a gene amplification system using a dihydrofolate reductase gene or the like according to the method described in Japanese Published Unexamined Patent Application No. 227075/90.

Moreover, the protein having α2,3/α2,8-sialyltransferase activity can be produced by rediferentiating an animal or plant cell into which a gene has been introduced to develop a gene-introduced transgenic animal individual (transgenic nonhuman animal) or plant individual (transgenic plant), and using the individual.

When the transformant is the animal individual or plant individual, the protein can be produced by breeding or cultivating it to produce and accumulate the protein, and recovering the protein from the animal individual or plant individual.

The process for producing the protein having α2,3/α2,8-sialyltransferase activity using the animal individual includes a method for producing the protein as disclosed in the context of the present invention in a nonhuman animal developed by introducing a gene according to a known method [Am. J. Clin. Nutr., 63, 639S (1996), Am. J. Clin. Nutr., 63, 627S (1996), Biol/Technology, 9, 830 (1991)].

In the animal individual, the protein, can be produced by breeding a transgenic nonhuman animal into which the DNA encoding the protein having α2,3/α2,8 sialyltransferase has been introduced to produce and accumulate the protein in the animal, and recovering the protein from the animal. The protein produced in the animal is accumulated in milk (Japanese Published Unexamined Patent Application No. 309192/88), egg, and the like. Any promoter can be used, so long as it can function in the animal. Suitable examples include an α-casein promoter, a β-casein promoter, a β-lactoglobulin promoter, a whey acidic protein promoter, and the like, which are specific for mammary glandular cells.

The process for producing the protein having α2,3/α2,8-sialyltransferase activity using the plant individual includes a process for producing the protein by cultivating a transgenic plant into which the DNA encoding the protein as disclosed in the context of the present invention is introduced by a known method [Tissue Culture (Soshiki Baiyo), 20 (1994), Tissue Culture (Soshiki Baiyo), 21 (1995), Trends Biotechnol., 15, 45 (1997)] to produce and accumulate the protein in the plant, and recovering the protein from the plant.

The protein having α2,3/α2,8-sialyltransferase activity produced by the production process of the protein as disclosed in the context of the present invention can be isolated and purified by using the general method for isolating and purifying an enzyme.

For example, when the protein having α2,3/α2,8-sialyltransferase activity is produced as a soluble product in the host cells, the cells are collected by centrifugation after culturing, suspended in an aqueous buffer, and disrupted using an ultrasonicator, a French press, a Manton Gaulin homogenizer, a Dynomill, or the like to obtain a cell-free extract.

From the supernatant obtained by centrifuging the cell-free extract, a purified product can be obtained by the general method used for isolating and purifying an enzyme, for example, solvent extraction, salting-out using ammonium sulfate or the like, desalting, precipitation using an organic solvent, anion exchange chromatography using a resin such as diethylaminoethyl (DEAE)-Sepharose or DIAION HPA-75 (manufactured by Mitsubishi Chemical), cation exchange chromatography using a resin such as S-Sepharose FF (manufactured by Pharmacia), hydrophobic chromatography using a resin such as butyl sepharose or phenyl sepharose, gel filtration using a molecular sieve, affinity chromatography, chromatofocusing, or electrophoresis such as isoelectronic focusing, alone or in combination thereof.

When the protein is produced as an inclusion body in the host cells, the cells are collected in the same manner, disrupted and centrifuged to recover the protein as the precipitate fraction, and then the inclusion body of the protein is solubilized with a protein-denaturing agent.

The solubilized solution is diluted or dialyzed in a solution free from a protein denaturing agent or a solution having a diluted concentration of a protein denaturing agent in such a degree that the protein is not denatured to thereby constitute the normal tertiary structure of the protein, and then a purified product of the protein can be obtained by a purification/isolation method similar to the above.

When the protein having α2,3/α2,8-sialyltransferase activity or derivatives such as its clycosylated-derivatives are secreted out of cells, the protein or its derivatives such as the glycosylated-derivatives can be collected in the culture supernatant.

Specifically, the culture medium is treated in a manner similar to the above, such as centrifugation to obtain a solubilized fraction, from which a purified product can be obtained using a purification/isolation method similar to the above.

The protein obtained by the above method includes a protein comprising the amino acid sequence represented by SEQ ID NO:1, 3 or 5.

Furthermore, the protein having α2,3/α2,8-sialyltransferase activity is produced as a fusion protein with other protein, and can be purified using affinity chromatography using a substance having affinity to the fusion protein. For example, the fusion protein having α2,3/α2,8-sialyltransferase activity is produced as a fusion protein with protein A according to the method of Lowe *et al.* [Proc. Natl. Acad. Sci. USA, 86, 8227 (1989); Genes Develop., 4, 1288 (1990)], or the method described in Japanese Published Unexamined Patent Application No. 336963/93 or WO94/23021 and the fusion protein can be purified by affinity chromatography using immunoglubulin G.

Moreover, the protein having α2,3/α2,8-sialyltransferase activity is produced as a fusion protein with Flag peptide, and the fusion protein can be purified by affinity chromatography using an anti-Flag antibody *[*Proc. Natl. Acad. Sci., USA, 86, 8227 (1989), Genes Develop., 4, 1288 (1990)]. In addition, purification can be carried out by affinity chromatography using the antibody against the polypeptide *per se.*

Based on the amino acid sequence information of the protein thus obtained, the protein having α2,3/α2,8-sialyltransferase can be produced by a chemical synthesis method, such as Fmoc (fluorenylmethyloxycarbonyl) method or tBoc (t-butyloxycarbonyl) method. It can also be chemically synthesized using a peptide synthesizer manufactured by Advanced ChemTech, Perkin-Elmer, Pharmacia, Protein Technology Instrument, Synthecell-Vega, PerSeptive, Shimadzu Corporation, or the like.

### (3) Preparation of sialic acid-containing complex carbohydrate

The process for producing a sialic acid-containing complex carbohydrate as disclosed in the context of the present invention is a process for production, which comprises: allowing a culture of a transformant capable of producing a protein having α2,3/α2,8-sialyltransferase activity derived from a microorganism or a treated product of the culture as an enzyme source, cytidine-5'-monophosphosialic acid and an acceptor complex carbohydrate to be present in an aqueous medium to produce and accumulate the sialic acid-containing complex carbohydrate in the aqueous medium, and recovering the sialic acid-containing complex carbohydrate from the aqueous medium.

The transformant used in the production process of the sialic acid-containing complex carbohydrate as disclosed in the context of the present invention is not particularly limited, so long as it is a transformant capable of producing a protein having α2,3/α2,8-sialyltransferase derived from a microorganism. The transformant is preferably a transformant comprising a DNA encoding a protein having α2,3/α2,8-sialyltransferase activity derived from a microorganism belonging to the genus *Pasteurella,* and more preferably a transformant comprising a DNA encoding the protein derived from *Pasteurella multocida.*

The DNA encoding the protein derived from *Pasteurella multocida* includes:
(1) a DNA encoding a protein comprising the amino acid sequence represented by SEQ ID NO:1;
(2) a DNA encoding a protein comprising the amino acid sequence represented by SEQ ID NO:3;
(3) a DNA encoding a protein comprising the amino acid sequence represented by SEQ ID NO: 5;
(4) a DNA comprising the nucleotide sequence represented by SEQ ID NO:2;
(5) a DNA comprising the nucleotide sequence represented by SEQ ID NO:4;
(6) a DNA comprising the nucleotide sequence represented by SEQ ID NO:6;
(7) a DNA encoding a protein which consists of an amino acid sequence in which at least one amino acid is deleted, substituted or added in the amino acid sequence represented by any one selected from SEQ ID NOs:1, 3 and 5, and has α2,3/α2,8-sialyltransferase activity; and
(8) a DNA which hybridizes with a DNA consisting of a complementary sequence of the nucleotide sequence represented by any one selected from SEQ ID NOs:2, 4 and 6 under stringent conditions, and encodes a protein having α2,3/α2,8-sialyltransferase activity.

The above-described DNA can be obtained by the method of the above item [1], and a transformant comprising the DNA and a culture of the transformant can be obtained by the method of the above item [2].

The treated product of the culture includes a concentrated product of the culture, a dried product of the culture, cells obtained by centrifuging the culture, a dried product of the cells, a freeze-dried product of the cells, a surfactant-treated product of the cells, an ultrasonic-treated product of the cells, a mechanically disrupted product of the cells, a solvent-treated product of the cells, an enzyme-treated product of the cells, a protein fraction of the cells, an immobilized product of the cells, an enzyme preparation obtained by extracting from the cell, and the like.

The enzyme source used in the production of the sialic acid-containing complex carbohydrate is used in a concentration of 1 mU/l to 1,000 U/l, preferably 10 mU/l to 500 U/l, when the activity capable of forming 1 µmol of sialic acid-containing complex carbohydrate at 37°C in 1 minute is defined as 1 unit (U).

The aqueous medium used in the production of the sialic acid-containing complex carbohydrate includes water; a buffer such as a phosphate buffer, a carbonate buffer, an acetate buffer, a borate buffer, a citrate buffer and a tris buffer; alcohol, such as methanol and ethanol; ester such as ethyl acetate; ketone such as acetone; amide such as acetamide; and the like. Also, the culture of the microorganisms used as the enzyme source can be used as an aqueous medium.

The acceptor complex carbohydrate used in the production of the sialic acid-containing complex carbohydrate includes a complex carbohydrate comprising an oligosaccharide having galactose at its non-reducing terminus and a complex carbohydrate comprising an oligosaccharide having sialic acid at its non-reducing terminus and is preferably a complex carbohydrate comprising an oligosaccharide having a structure selected from the group consisting of lactose, globotriose, *N-*acetyllactosamine, lacto-N-tetraose, lacto-*N*-neotetraose, Lewis^{a} and Lewis^{x} in its non-reducing terminal or a complex carbohydrate comprising an oligosaccharide having a structure selected from the group consisting of NeuAcα2-3Galβ1-4Glc and NeuAcα2-3Galβ1-4GlcNAc at its non-reducing terminus.

In the production of the sialic acid-containing complex carbohydrate, a surfactant or an organic solvent may be added, if necessary. Any surfactant capable of accelerating the formation of a sialic acid-containing complex carbohydrate can be used as the surfactant. Examples include nonionic surfactants such as polyoxyethylene octadecylamine (e.g., Nymeen S-215, manufactured by Nippon Oil & Fats); cationic surfactants, such as cetyltrimethylammonium bromide and alkyldimethyl benzylammoniumchloride (e.g., Cation F2-40E, manufactured by Nippon Oil & Fats); anionic surfactants such as lauroyl sarcosinate; tertiary amines such as alkyldimethylamine (e.g., Tertiary Amine FB, manufactured by Nippon Oil & Fats); and the like, which are used alone or as a mixture of two or more. The surfactant is used generally in a concentration of 0.1 to 50 g/l. The organic solvent includes xylene, toluene, fatty acid alcohol, acetone, ethyl acetate, and the like, which are used in a concentration of generally 0.1 to 50 ml/l.

The production reaction for the sialic acid-containing complex carbohydrate is carried out in an aqueous medium having a pH 5 to 10, preferably pH 6 to 8, at 20 to 50°C for 1 to 96 hours. In the production reaction, inorganic salts, such as MnCl₂ and MgCl₂, can be added, if necessary.

The amount of the sialic acid-containing complex carbohydrate produced in the aqueous medium can be determined, for, example, using a carbohydrate analysis system manufactured by Dionex [Anal. Biochem., 189:, 151 (1990)] or the like.

The sialic acid-containing complex carbohydrate produced in the aqueous medium can be recovered by the ordinary method using activated carbon, an ion exchange resin or the like.

Examples of the present invention are shown below; however, the present invention is not limited to these Examples.

### BRIEF DESCRIPTION OF THE DRAWING

Fig. 1 shows construction steps of an α2,3/α2,8-sialyltransferase gene expression plasmid pPT76.
Fig. 2 shows construction steps of an α2,3/α2,8-sialyltransferase gene expression plasmid pPT79.
Fig.3 shows construction steps of an α2,3/α2,8-sialyltransferase gene expression plasmid pPT78.

Also, in the drawings, Amp^{r} represents an ampicillin-resistant gene; P_{L} represents P_{L} promoter; cI857 represents a temperature-sensitive repressor gene; pm0188 represents a gene encoding an α2,3/α2,8-siatyltransferase; pm0508 represents a gene encoding an α2,3/α2,8-sialyltransferase; and pm1174 represents a gene encoding an α2,3/α2,8-sialyltransferase.

### BEST MODE FOR CARRYING OUT THE INVENTION

### Example 1 Construction of a strain expressing a gene encoding an α2,3/α2,8-sialyltransferase derived from Pasteurella multocida

### (1) Construction of Escherichia coli NM522/pPT76

A chromosomal DNA of *Pasteurella multocida* PM70 was obtained from Minnesota University.

Using DNA fragments having the nucleotide sequences represented by SEQ ID NOs:7 and 8 which had been synthesized by using a DNA synthesizer Model 8905 manufactured by Perseptive Biosystems, a DNA fragment containing pm0188 considered to be a gene encoding an α2,6-sialyltransferase in the full nucleotide sequence of the genomic DNA in *Pasteurella multocida* PM70 was amplified by the following method.

PCR was carried out by using the above synthetic DNA fragments as a primer set and using the chromosomal DNA of *Pasteurella multocida* PM70 as the template. The PCR was carried out by using 40 µl of a reaction solution containing 0.1 µg of the chromosomal DNA, 0.5 µmol/l of each of the primers, 2.5 units of Pfu DNA polymerase (manufactured by Stratagene), 4 µl of 10 x buffer for Pfu DNA polymerase and 200 µmol/l of each deoxy NTP, and repeating 30 times of a cycle consisting of 1 minute at 94°C, 2 minutes at 42°C and 3 minutes at 72°C.

A 1/10 volume of the reaction solution was subjected to agarose gel electrophoresis to confirm that the fragment of interest was amplified, and then the remaining reaction solution was mixed with the same volume of TE [10 mmol/l Tris-HCl and 1 mmol/l EDTA (pH 8.0)] saturated phenol/chloroform (1 vol/1 vol).

After centrifugation of the mixed solution, the thus obtained upper layer was mixed with 2 volumes of cold ethanol and allowed to stand at -80°C for 30 minutes. A precipitate of DNA was obtained by centrifuging the solution.

The DNA precipitate was dissolved in 20 µl ofTE.

Using 5 µl of the dissolved solution, the DNA was digested with restriction enzymes *Cla*I and *Bam*HI, the resulting DNA fragments were separated by agarose gel electrophoresis, and then a DNA fragment of 1.2 kb was recovered by using Gene Clean II Kit (purchased from Funakoshi).

After 0 2 µg of pBluescript II SK(+) was digested with restriction enzymes *Cla*I and *Bam*HI, the resulting DNA fragments were separated by agarose gel electrophoresis, and then a DNA fragment of 2.9 kb was recovered in the same manner.

Using a ligation kit (manufactured by Takara Shuzo), the 1.2 kb and 2.9 kb fragments were subjected to ligation reaction at 16°C for 16 hours. *Escherichia coli* NM522 was transformed by using the ligation reaction solution in accordance with the above known method, and the transformant was spread on an LB agar medium [10 g/l Bacto-Tryptone (manufactured by Difco), 10 g/l yeast extract (manufactured by Difco), 5 g/l sodium chloride and 15 g/l agarose] containing 50 µg/ml ampicillin, followed by culturing overnight at 30°C.

A plasmid was extracted from the thus formed transformant colonies in accordance with the above known method, the structure of the plasmid was confirmed by restriction enzyme digestion, and the plasmid was named pPMO188SK. The plasmid pPMO188SK has been deposited on September 13, 2001, as FERM BP-7730 in International Patent Organism Depositary, National Institute of Advanced Industrial Science and Technology (Central 6, 1-1, Higashi 1-Chome Tsukuba, Ibaraki, 305-8566, Japan).

Next, pPMO188SK was digested with restriction enzymes *Cla*I and *Bam*HI, the resulting DNA fragments were separated by agarose gel electrophoresis, and then a DNA fragment of 1.2 kb was recovered. After 0.2 µg of pPAC31 DNA was digested with restriction enzymes *Cla*I and *Bam*HI, the resulting DNA fragments were separated by agarose gel electrophoresis, and then a DNA fragment of 5.4 kb was recovered in the same manner.

Using the ligation kit, the 1.2 kb and 5.4 kb fragments were subjected to ligation reaction at 16°C for 16 hours.

*Escherichia coli* NM522 was transformed by using the ligation reaction solution in accordance with the above known method, and the transformant was spread on the LB agar medium containing 50 µg/ml ampicillin, followed by culturing overnight at 30°C.

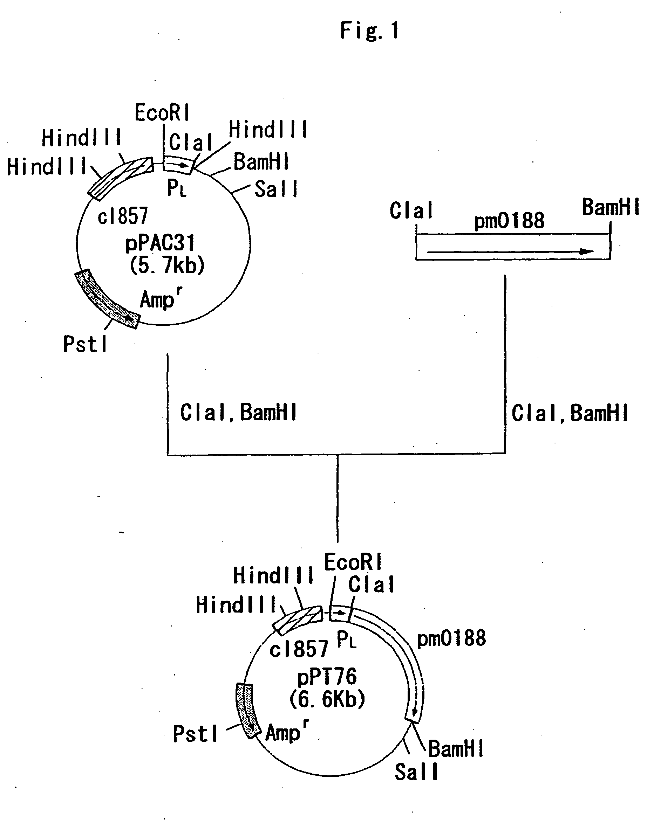

A plasmid was extracted from the thus formed transformant colonies in accordance with the above known method to thereby obtain the expression plasmid pPT76. The structure of the plasmid was confirmed by restriction enzyme digestion (Fig. 1).

### (2) Construction of Escherichia coli NM522/pPT79

In the same manner as in the above item (1), a DNA of interest was obtained by PCR in the following manner by using a chromosomal DNA of *Pasteurella multocida* PM70.

Using DNA fragments having the nucleotide sequences represented by SEQ ID NOs:9 and 10 which had been synthesized by using a DNA synthesizer Model 8905 manufactured by Perseptive Biosystems, a DNA fragment containing pm508 considered to be a gene encoding an α2,3-sialyltransferase in the full nucleotide sequence of the genomic DNA in *Pasteurella multocida* PM70 was amplified by the following method.

PCR was carried out by using the above synthetic DNA fragments as a primer set and using the chromosomal DNA of *Pasteurella multocida* PM70 as the template. The PCR was carried out by using 40 µl of a reaction solution containing 0.1 µg of the chromosomal DNA, 0.5 µmol/l of each of the primers, 2.5 units of Pfu DNA polymerase (manufactured by Stratagene), 4 µl of 10 x buffer for Pfu DNA polymerase and 200 µmol/l of each deoxy NTP, and repeating 30 times of a cycle consisting of 1 minute at 94°C, 2 minutes at 42°C and 3 minutes at 72°C.

A 1/10 volume of the reaction solution was subjected to agarose gel electrophoresis to confirm that the fragment of interest was amplified, and then the remaining reaction solution was mixed with the same volume of TE [10 mmol/l Tris-HCl and 1 mmol/l EDTA (pH 8.0)] saturated phenol/chloroform (1 vol/1 vol).

After the mixed solution was centrifuged, the thus obtained upper layer was mixed with 2 volumes of cold ethanol and allowed to stand at -80°C for 30 minutes. A precipitate of DNA was obtained by centrifuging the solution.

The DNA precipitate was dissolved in 20 µl of TE.

Using 5 µl of the dissolved solution, the DNA was digested with restriction enzymes *Cla*I and *Bam*HI*,* the resulting DNA fragments were separated by agarose gel electrophoresis, and then a DNA fragment of 1.0 kb was recovered by using the Gene Clean II Kit.

After 0.2 µg of pBluescript II SK(+) was digested with restriction enzymes *Cla*I and *Bam*HI, the resulting DNA fragments were separated by agarose gel electrophoresis, and then a DNA fragment of 2.9 kb was recovered in the same manner.

Using the ligation kit, the 1.0 kb and 2.9 kb fragments were subjected to libation reaction at 16°C for 16 hours. *Escherichia coli* NM522 was transformed by using the ligation reaction solution in accordance with the above known method, and the transformant was spread on the LB agar medium containing 50 µg/ml ampicillin, followed by culturing overnight at 30°C.

A plasmid was extracted from the thus formed transformant colonies in accordance with the above known method, the structure of the plasmid was confirmed by restriction enzyme digestion, and the plasmid was named pPMO508SK. The plasmid pPMO508SK has been deposited on September 13, 2001, as FERM BP-7731 in International Patent Organism Depositary, National Institute of Advanced Industrial Science and Technology (Central 6, 1-1, Higashi 1-Chome Tsukuba, Ibaraki, 305-8566, Japan).

Next, pPMO508SK was digested with restriction enzymes *Cla*I and *Bam*HI, the resulting DNA fragments were separated by agarose gel electrophoresis, and then a DNA fragment of 1.0 kb was recovered. After 0.2 µg of pPAC31 DNA was digested with restriction enzymes *Cla*I and *Bam*HI*,* the resulting DNA fragments were separated by agarose gel electrophoresis, and then a DN.4 fragment of 5.4 kb was recovered in the same manner.

Using the ligation kit, the 1.0 kb and 5.4 kb fragments were subjected to ligation reaction at 16°C for 16 hours.

*Escherichia coli* NM522 was transformed by using the ligation reaction solution in accordance with the above known method, and the transformant was spread on the LB agar medium containing 50 µg/ml ampicillin, followed by culturing overnight at 30°C.

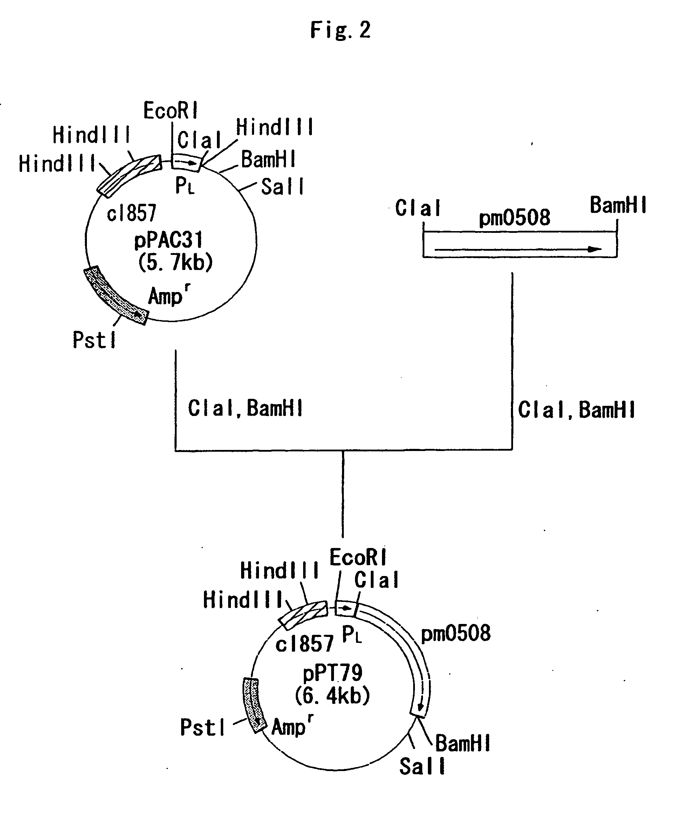

A plasmid was extracted from the thus formed transformant colonies in accordance with the above known method to thereby obtain the expression plasmid pPT79. The structure of the plasmid was confirmed by restriction enzyme digestion (Fig. 2).

### (3) Construction of Escherichia coli NM522/pPT78

In the same manner as in the above item (1, a DNA of interest was obtained by PCR using chromosomal DNA of *Pasteurella multocida* PM70.

Using DNA fragments having the nucleotide sequences represented by SEQ ID NOs:11and 12 which had been synthesized by using a DNA synthesizer Model 8905 manufactured by Perseptive Biosystems, a DNA fragment containing pm1174 considered to be a gene encoding an α,3-sialyltransferase in the full nucleotide sequence of the genomic DNA in *Pasteurella multocida* PM70 was amplified by the following method.

PCR was carried out by using the above synthetic DNA fragments as a primer set and using the chromosomal DNA of *Pasteurella multocida* PM70 as the template. The PCR was carried out by using 40 µl of a reaction solution containing 0.1 µ of the chromosomal DNA, 0.5 µmol/l of each of the primers, 2.5 units of Pfu DNA polymerase (manufactured by Stratagene), 4 µl of 10 x buffer for Pfu DNA polymerase and 200 µmol/l of each deoxy NTP, and repeating 30 times of a cycle consisting of 1 minute at 94°C, 2 minutes at 42°C and 3 minutes at 72°C.

A 1/10 volume of the reaction solution was subjected to agarose gel electrophoresis to confirm that the fragment of interest was amplified, and then the remaining reaction solution was mixed with the same volume of TE [10 mmol/l Tris-HCl and 1 mmol/l EDTA (pH 8.0)] saturated phenol/chloroform (1 vol/1 vol).

After centrifugation of the mixed solution, the thus obtained upper layer was mixed with 2 volumes of cold ethanol and allowed to stand at -80°C for 30 minutes. A precipitate of DNA was obtained by centrifuging the solution.

The DNA precipitate was dissolved in 20 µl of TE.

Using 5 µl of the dissolved solution, the DNA was digested with restriction enzymes *Cla*I and *Bam*HI*,* the resulting DNA fragments were separated by agarose gel electrophoresis, and then a DNA fragment of 0.9 kb was recovered by using Gene Clean 11 Kit (purchased from Funakoshi).

After 0.2 µg of pBluescript II SK(+) was digested with restriction enzymes *Cla*I and *Bam*HI*,* the resulting DNA fragments were separated by agarose gel electrophoresis, and then a DNA fragment of 2.9 kb was recovered in the same manner.

Using the ligation kit, the 0.9 kb and 2.9 kb fragments were subjected to ligation reaction at 16°C for 16 hours. *Escherichia coli* NM522 was transformed by using the ligation reaction solution in accordance with the above known method, and the transformant was spread on the LB agar medium containing 50 µg/ml ampicillin, followed by culturing overnight at 30°C..

A plasmid was extracted from the thus formed transformant colonies in accordance with the above known method, the structure of the plasmid was confirmed by restriction enzyme digestion, and the plasmid was named pPM1174SK. The plasmid pPM1174SK has been deposited on September 13, 2001, as FERM BP-7733 in International Patent Organism Depositary, National Institute of Advanced Industrial Science and Technology (Central 6, 1-1, Higashi 1-Chome Tsukuba, Ibaraki, 305-8566, Japan).

Next, pPM1174SK was digested with restriction enzymes *Cla*I and *Bam*HI*,* the resulting DNA fragments were separated by agarose gel electrophoresis, and then a DNA fragment of 0.9 kb was recovered. After 0.2 µg of pPAC31 DNA was digested with restriction enzymes *Cla*I and *Bam*HI*,* the resulting DNA fragments were separated by agarose gel electrophoresis, and then a DNA fragment of 5.4 kb was recovered in the same manner.

Using the ligation kit, the 0.9 kb and 5.4 kb fragments were subjected to ligation reaction at 16°C for 16 hours.

*Escherichia coli* NM522 was transformed by using the ligation reaction solution in accordance with the above known method, and the transformant was spread on the LB agar medium containing 50 µg/ml ampicillin, followed by culturing overnight at 30°C.

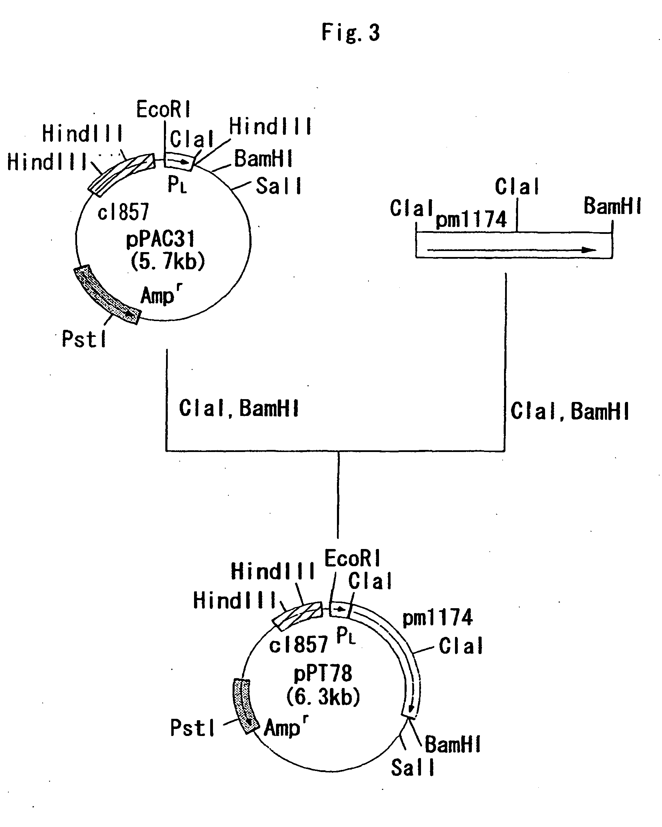

A plasmid was extracted from the thus formed transformant colonies in accordance with the above known method to thereby obtain the expression plasmid pPT78. The structure of the plasmid was confirmed by restriction enzyme digestion (Fig. 3).

### Example 2 Production of a sialic acid-containing complex carbohydrates using Escherichia coli NM522/pPT76

### (1) Production of NeuAcα2-3Galβ1-4Glc

*Escherichia coli* NM522/pPT76 obtained in the item (1) of Example 1 was inoculated into a test tube charged with 8 ml of LB medium containing 50 µg/ml ampicillin, followed by culturing at 28°C for 17 hours. The culture was inoculated into a test tube charged with 8 ml of LB medium containing 50 µg/ml ampicillin, with an inoculum size of 1%, followed by culturing, at 28°C for 2 hours and then at 40°C for 3 hours. Wet cells were obtained by centrifuging 0.5 ml of the culture. The wet cells can be stored at -20°C, if necessary, and it was possible to use them by thawing prior to use.

The reaction was carried out at 37°C for 16 hours in 0.1 ml of a reaction solution containing the NM522/pPT76 wet cells obtained in the above. 50 mmol/l citrate buffer (pH 7.0), 5 mmol/l MnCl₂, 10 mmol/l lactose, 5 mmol/l CMP-sialic acid and 4 g/l Nymeen S-215.

After completion of the reaction, the reaction product was analyzed by using a sugar analyzer manufactured by Dionex (DX-500) under the following analyzing conditions to confirm that 2.69 mmol/l (1,704 mg/l) NeuAcα2-3Galβ1-4Glc was formed and accumulated in the reaction solution.

### Analyzing conditions:

- Column:: CarboPAC PA10 (manufactured by Dionex)
- Eluent:: eluent A; 500 mmol/l NaOH, eluent B; 100 mmol/l NaOH + 500 mmol/l NaOAc
- Gradient:: Linear gradient from a composition comprising 20% of the eluent A and 10% of the eluent B at 0 minute to a composition comprising 30% of the eluent A and 25% of the eluent B spending 30 minutes.
- Detector:: Pulsed amperometry detector

### (2) Production of NeuAcα2-3Galβ1-4GlcNAcβ1-3Galβ1-4Glc

*Escherichia coli* NM522/pPT76 obtained in the item (1) of Example 1(1) was inoculated into a test tube charged with 8 ml of LB medium containing 50 µg/ml ampicillin, followed by culturing, at 28°C for 17 hours. The culture was inoculates into a test tube charged with 8 ml of LB medium containing 50 µg/ml ampicillin, with an inoculum size of 1.%, followed by culturing at 28°C for 2 hours and then at 40°C for 3 hours. Wet cells were obtained by centrifuging 0.5 ml of the culture. The wet cells can be stored at -20°C, if necessary, and it was possible to use them by thawing prior to use.

The reaction was carried out at 37°C for 16 hours in 0.1 ml of a reaction solution containing the NM522/pPT76 wet cells obtained in the above, 50 mmol/l citrate buffer (pH 7.0), 5 mmol/l MnCl₂, 10 mmol/l lacto-N-neotetraose, 5 mmol/l CMP-sialic acid and 4 g/l Nymeen S-215.

After completion of the reaction, the reaction product was analyzed by using a sugar analyzer manufactured by Dionex (DX-500) under the analyzing conditions described in the item (1) of Example 2 to confirm that 1.64 mmol/l (1,638 mg/l) NeuAcα2-3Galβ1-4GlcNacβ1-3Galβ1-4Glc was formed and accumulated in the reaction solution.

### (3) Production of NeuAcα2-8NeuAcα2-3Galβ1-4Glc

*Escherichia coli* NM522/pPT76 obtained in the item (1) of Example 1 was inoculated into a thick test tube charged with 8 ml of LB medium containing 50 µg/ml ampicillin, followed by culturing at 28°C for 17 hours. The culture was inoculated into a thick test tube charged with 8 ml of LB medium containing 50 µg/ml ampicillin, with an inoculum size of 1%, followed by culturing at 28°C for 2 hours and then at 40°C for 3 hours. Wet cells were obtained by centrifuging 0.5 ml of the culture. The wet cells can be stored at -20°C, if necessary, and it was possible to use them by thawing prior to use.

The reaction was carried out at 37°C for 2 hours in 0.1 ml of a reaction solution containing the NM522/pPT76 wet cells obtained in the above, 50 mmol/l citrate buffer (pH 7.0), 5 mmol/l MnCl₂, 2 mmol/l NeuAcα2-3Galβ1-4Glc, 2 mmol/l CMP-sialic acid and 4 g/l Nymeen S-215.

After completion of the reaction, the reaction product was analyzed by using a sugar analyzer manufactured by Dionex (DX-500) under the following analyzing conditions to confirm that 0.43 mmol/l (397 mg/l) NeuAcα2-8NeuAcα2-3Galβ1-4Glc was formed and accumulated in the reaction solution.

### Analyzing conditions:

- Column:: CarboPAC PA10
- Eluent:: eluent A; H₂O, eluent B; 500 mmol/l NaOH, eluent C; 100 mmol/l NaOH + 500 mmol/l NaOAc
- Gradient:: Linear gradient from a composition comprising 10% of the eluent A, 60% of the eluent B and 30% of the eluent C at 0 minute to a composition comprising 0% of the eluent A, 65% of the eluent B and 35% of the eluent C spending 15 minutes.
- Detector:: Pulsed amperometry detector

### Example 3 Production of sialic acid-containing complex carbohydrates using Escherichia coli NM522/pPT79

### (1) Production of NeuAcα2-3Galβ1-4Glc

*Escherichia coli* NM522/pPT79 obtained in the item (2) of Example 1 was inoculated into a test tube charged with 8 ml of LB medium containing 50 µg/ml ampicillin, followed by culturing at 28°C for 17 hours. The culture was inoculated into a test tube charged with 8 ml of LB medium containing 50 µg/ml ampicillin, with an inoculum size of 1%, followed by culturing at 28°C for 2 hours and then at 40°C for 3 hours. Wet cells were obtained by centrifuging 0.5 ml of the culture. The wet cells can be stored at -20°C, if necessary, and it was possible to use them by thawing prior to use.

The reaction was carried out at 37°C for 6 hours in 0.1 ml of a reaction solution containing the NM522/pPT79 wet cells obtained in the above, 50 mmol/l citrate buffer (pH 7.0), 5 mmol/l MnCl₂, 10 mmol/l lactose, 5 mmol/l CMP-sialic acid and 4 g/l Nymeen S-215.

After completion of the reaction, the reaction product was analyzed by using a sugar analyzer manufactured by Dionex (DX-500) under the analyzing conditions described in the item (1) of Example 2 to confirm that 0.25 mmol/l (164 mg/l) NeuAcα2-3Galβ1-4Glc was formed and accumulated in the reaction solution.

### (2) Production of NeuAcα2-8NeuAcα2-3Galβ1-4Glc

*Escherichia coli* NM522/pPT79 obtained in the item (2) of Example 1 was inoculated into a thick test tube charged with 8 ml of LB medium containing 50 µg/ml ampicillin, followed by culturing at 28°C for 17 hours. The culture was inoculated into a thick test tube charged with 8 ml of LB medium containing 50 µg/ml ampicillin, with an inoculum size of 1%, followed by culturing at 28°C for 2 hours and then at 40°C for 3 hours. Wet cells were obtained by centrifuging 0.5 ml of the culture. The wet cells can be stored at -20°C, if necessary, and it was possible to use them by thawing prior to use.

The reaction was carried out at 37°C for 2 hours in 0.1 ml of a reaction solution containing the NM522/pPT79 wet cells obtained in the above, 50 mmol/l citrate buffer (pH 7.0), 5 mmol/l MnCl₂, 2 mmol/l NeuAcα2-3Galβ1-4Glc, 2 mmol/l CMP-sialic acid and 4 g/l Nymeen S-215.

After completion of the reaction, the reaction product was analyzed by using a sugar analyzer manufactured by Dionex (DX-500) under the analyzing conditions described in the item (3) of Example 2 to confirm that 0.54 mmol/l (499 mg/l) NeuAcα2-8NeuAcα2-3Galβ1-4Glc was formed and accumulated in the reaction solution.

### Example 4 Production of sialic acid-containing complex carbohydrates using Escherichia coli NM522/pPT78

### (1) Production of NeuAcα2-3Galβ1-4Glc

*Escherichia coli* NM522/pPT78 obtained in the item (3) of Example 1 was inoculated into a test tube charged with 8 ml of LB medium containing 50 µg/ml ampicillin, followed by culturing at 28°C for 17 hours. The culture was inoculated into a test tube charged with 8 ml of LB medium containing 50 µg/ml ampicillin, with an inoculum size of 1%, followed by culturing at 28°C for 2 hours and then at 40°C for 3 hours. Wet cells were obtained by centrifuging 0.5 ml of the culture. The wet cells can be stored at -20°C, if necessary, and it was able to use them by thawing prior to use.

The reaction was carried out at 37°C for 16 hours in 0.1 ml of a reaction solution containing the NM522/pPT78 wet cells obtained in the above, 50 mmol/l citrate buffer (pH 7.0), 5 mmol/l MnCl₂, 10 mmol/l lactose, 5 mmol/l CMP-sialic acid and 4 g/l Nymeen S-215.

After completion of the reaction, the reaction product was analyzed using a sugar analyzer manufactured by Dionex (DX-500) under the analyzing conditions described in the item (1) of Example 2 to confirm that 0.98 mmol/l (621 mg/l) NeuAcα2-3Galβ1-4Glc was formed and accumulated in the reaction solution:

### (2) Production of NeuAcα2-8NeuAcα2-3Galβ1-4Glc

*Escherichia coli* NM522/pPT78 obtained in the item (3) of Example 1 was inoculated into a test tube charged with 8 ml of LB medium containing 50 µg/ml ampicillin, followed by culturing at 28°C for 17 hours. The culture was inoculated into a test tube charged with 8 ml of LB medium containing 50 µg/ml ampicillin, with an inoculum size of 1%, followed by culturing at 28°C for 2 hours and then at 40°C for 3 hours. Wet cells were obtained by centrifuging 0.5 ml of the culture. The wet cells can be stored at -20°C, if necessary, and it was possible to use them by thawing prior to use.

The reaction was carried out at 37°C for 2 hours in 0.1 ml of a reaction solution containing the NM522/pPT78 wet cells obtained in the above, 50 mmol/l citrate buffer (pH 7.0), 5 mmol/l MnCl₂, 2 mmol/l NeuAcα2-3Galβ1-4Glc, 2 mmol/l CMP-sialic acid and 4 g/l Nymeen S-215.

After completion of the reaction, the reaction product was analyzed by using a sugar analyzer manufactured by Dionex (DX-500) under the analyzing conditions described in the item (3) of Example 2 to confirm that 0.43 mmol/l (397 mg/l) NeuAcα2-8NeuAcα2-3Galβ1-4Glc was formed and accumulated in the reaction solution.

### INDUSTRIAL APPLICABILITY

According to the present invention, an α2,3/α2,8-sialyltransferase can be produced in a large amount. Also, a sialic acid-containing complex carbohydrate can be produced efficiently by using the enzyme.

### Free Text of Sequence Listing:

SEQ ID NO:7 - Description of artificial sequence: Synthetic DNA
SEQ ID NO:8. Description of artificial sequence: Synthetic DNA
SEQ ID NO:9 - Description of artificial sequence: Synthetic DNA
SEQ ID NO:10 - Description of artificial sequence: Synthetic DNA.
SEQ ID NO:11 - Description of artificial sequence: Synthetic DNA
SEQ ID NO:12 - Description of artificial sequence: Synthetic DNA

## Claims

1. A process for producing a sialic acid-containing complex carbohydrate, which comprises: allowing a culture of a transformant capable of producing a protein having α2,3/α2,8-sialyltransferase activity derived from a microorganism or a treated product of the culture as an enzyme source, cytidine-5'-monophosphosialic acid and an acceptor complex carbohydrate to be present in an aqueous medium to produce and accumulate the sialic acid-containing complex carbohydrate in the aqueous medium, and recovering the sialic acid-containing complex carbohydrate from the aqueous medium,
wherein the protein having α2,3/α2,8-sialyltransferase activity is a protein selected from the group consisting of:
(a) a protein comprising the amino acid sequence represented by SEQ ID NO: 1;
(b) a protein which consists of an amino acid sequence in which 1 to 20 amino acids are deleted, substituted or added in the amino acid sequence represented by SEQ ID NO: 1, and has α2,3/α2,8-sialyltransferase activity; and
(c) a protein which has an identity of at least 80% with the amino acid sequence represented by SEQ ID NO: 1, and has α2,3/α2,8-sialyltransferase activity.

2. The process according to claim 1, wherein the treated product of the culture is selected from the group consisting of a concentrated product of the culture, a dried product of the culture, cells obtained by centrifuging the culture, a dried product of the cells, a freeze-dried product of the cells, a surfactant-treated product of the cells, an ultrasonic-treated product of the cells, a mechanically disrupted product of the cells, a solvent-treated product of the cells, an enzyme-treated product of the cells, a protein fraction of the cells, an immobilized product of the cells and an enzyme preparation obtained by extracting from the cells.

3. The process according to claim 1, wherein the acceptor complex carbohydrate is a complex carbohydrate comprising an oligosaccharide having galactose in its non-reducing terminal.

4. The process according to claim 1, wherein the acceptor complex carbohydrate is a complex carbohydrate comprising an oligosaccharide having sialic acid in its non-reducing terminal.

5. The process according to claim 3, wherein the oligosaccharide having galactose in its non-reducing terminal is an oligosaccharide selected from the group consisting of lactose, globotriose, N-acetyllactosamine, lacto-N-tetraose, lacto-N-neotetraose, Lewis^{a} and Lewis^{x}.

6. The process according to claim 4, wherein the oligosaccharide having sialic acid in its non-reducing terminal is an oligosaccharide selected from the group consisting of NeuAcα2-3Galβ1-4Glc and NeuAcα2-3Galβ1-4GlcNAc.

7. The process according to claim 1, wherein the microorganism is a microorganism belonging to the genus *Pasteurella.*

8. The process according to claim 7, wherein the microorganism belonging to the genus *Pasteurella* is *Pasteurella multocida.*

9. The process according to claim 1, wherein the transformant is a transformant which comprises a recombinant DNA comprising a DNA encoding said protein having α2,3/α2,8-sialyltransferase activity.

10. The process according to claim 9, wherein the transformant is obtained by using a microorganism as a host cell.

11. The process according to claim 10, wherein the microorganism is *Escherichia coli.*

12. The process according to claim 9, wherein the DNA encoding the protein having α2,3/α2,8-sialyltransferase activity is
(a) a DNA comprising the nucleotide sequence represented by SEQ ID NO: 2; or
(b) a DNA which hybridizes with a DNA consisting of a complementary sequence of the nucleotide sequence represented by SEQ ID NO: 2 under stringent conditions, and encodes a protein having α2,3/α2,8-sialyltransferase activity.

## Patentansprüche

1. Verfahren zur Herstellung eines Sialinsäure enthaltenden komplexen Kohlenhydrats, das umfasst: Ermöglichen des Vorliegens einer Transformantenkultur, die imstande ist, ein Protein herzustellen, das eine von einem Mikroorganismus stammende α2,3/α2,8-Sialyltransferaseaktivität hat, oder eines aufbereiteten Produkts der Kultur als eine Enzymquelle, von Cytidin-5'-monophosphosialinsäure und einem komplexen Kohlenhydrat als Akzeptor in einem wässrigen Medium, um das Sialinsäure enthaltende komplexe Kohlenhydrat im wässrigen Medium herzustellen und anzureichern, und Gewinnen des Sialinsäure enthaltenden komplexen Kohlenhydrats aus dem wässrigen Medium,
wobei das Protein, das α2,3/α2,8-Sialyltransferaseaktivität hat, ein Protein ist ausgewählt aus der Gruppe bestehend aus:
(a) einem Protein umfassend die in SEQ ID NO:1 gezeigte Aminosäuresequenz;
(b) einem Protein, das aus einer Aminosäuresequenz besteht, in der 1 bis 20 Aminosäuren in der in SEQ ID NO: 1 gezeigten Aminosäuresequenz deletiert, substituiert oder hinzugefügt sind, und das eine α2,3/α2,8-Sialyltransferaseaktivität hat; und
(c) einem Protein, das eine Identität von mindestens 80% mit der in SEQ ID NO:1 gezeigten Aminosäuresequenz hat, und das eine α2,3/α2,8-Sialyltransferaseaktivität hat.

2. Verfahren nach Anspruch 1, wobei das aufbereitete Kulturprodukt ausgewählt ist aus der Gruppe bestehend aus einem konzentrierten Kulturprodukt, einem getrockneten Kulturprodukt, Zellen, die durch Zentrifugieren der Kultur erhalten wurden, einem getrockneten Produkt der Zellen, einem gefriergetrockneten Produkt der Zellen, einem mit einem oberflächenaktiven Stoff behandelten Produkt der Zellen, einem ultraschallbehandelten Produkt der Zellen, einem mechanisch aufgebrochenen Produkt der Zellen, einem lösungsmittelbehandelten Produkt der Zellen, einem enzymbehandelten Produkt der Zellen, einer Proteinfraktion der Zellen, einem immobilisierten Produkt der Zellen und einer Enzymaufbereitung, die durch Extraktion aus den Zellen erhalten wurde.

3. Verfahren nach Anspruch 1, wobei das komplexe Kohlenhydrat als Akzeptor ein komplexes Kohlenhydrat ist, das ein Oligosaccharid umfasst, das Galactose an seinem nicht-reduzierenden Ende hat.

4. Verfahren nach Anspruch 1, wobei das komplexe Kohlenhydrat als Akzeptor ein komplexes Kohlenhydrat ist, das ein Oligosaccharid umfasst, das Sialinsäure an seinem nicht-reduzierenden Ende hat.

5. Verfahren nach Anspruch 3, wobei das Oligosaccharid, das Galactose am nicht-reduzierenden Ende hat, ein Oligosaccharid ist, ausgewählt aus der Gruppe bestehend aus Lactose, Globotriose, *N-*Acetyllactosamin, Lacto-*N-*tetraose, Lacto-*N*-neotetraose, Lewis^{a} und Lewis^{x}.

6. Verfahren nach Anspruch 4, wobei das Oligosaccharid, das Sialinsäure am nicht-reduzierenden Ende hat, ein Oligosaccharid ist, ausgewählt aus der Gruppe bestehend aus NeuAcα2-3Galβ1-4Glc und NeuAca2-3Galβ1-4GlcNAc.

7. Verfahren nach Anspruch 1, wobei der Mikroorganismus ein Mikroorganismus ist, der zur Gattung *Pasteurella* gehört.

8. Verfahren nach Anspruch 7, wobei der Mikroorganismus, der zur Gattung *Pasteurella* gehört, *Pasteurella multocida* ist.

9. Verfahren nach Anspruch 1, wobei die Transformante eine Transformante ist, umfassend eine rekombinante DNA, die eine DNA umfasst, die das Protein mit α2,3/α2,8-Sialyltransferaseaktivität codiert.

10. Verfahren nach Anspruch 9, wobei die Transformante durch Verwendung eines Mikroorganismus als Wirtszelle erhalten wird.

11. Verfahren nach Anspruch 10, wobei der Mikroorganismus *Escherichia coli* ist.

12. Verfahren nach Anspruch 9, wobei die DNA, die das Protein mit α2,3/α2,8-Sialyltransferaseaktivität codiert, ist:
(a) eine DNA umfassend die in SEQ ID NO:2 gezeigte Nucleotidsequenz; oder
(b) eine DNA, die unter stringenten Bedingungen mit einer DNA hybridisiert, die aus einer komplementären Sequenz der in SEQ ID NO:2 gezeigten Nucleotidsequenz besteht und ein Protein codiert, das α2,3/α2,8-Sialyltransferaseaktivität hat.

## Revendications

1. Procédé de production d'un glucide complexe contenant de l'acide sialique, lequel comprend le fait de: permettre la mise en présence, en milieu aqueux, d'une culture d'un organisme génétiquement modifié capable de produire une protéine présentant une activité α2,3/α2,8-sialyltransférase provenant d'un microorganisme, ou d'un produit traité de la culture, comme source d'enzyme, de cytidine-5'-monophosphate-acide sialique et d'un glucide complexe accepteur, afin de produire et d'accumuler le glucide complexe contenant de l'acide sialique dans le milieu aqueux, et de recouvrer le glucide complexe contenant de l'acide sialique à partir du milieu aqueux,
la protéine qui présente une activité α2,3/α2,8-sialyltransférase étant une protéine choisie dans le groupe comprenant :
(a) une protéine qui comprend la séquence d'acides aminés présentée en tant Séquence n°1 ;
(b) une protéine qui comprend une séquence d'acides aminés dans laquelle 1 à 20 acides aminés sont supprimés, substitués ou ajoutés dans la séquence d'acides aminés présentée en tant Séquence n° 1, et possède une activité α2,3/α2,8-sialyltransférase ; et
(c) une protéine qui présente une identité d'au moins 80% avec la séquence d'acides aminés présentée en tant que Séquence n° 1, et qui possède une activité α2,3/α2,8-sialyltransférase.

2. Procédé selon la revendication 1, le produit transformé de la culture étant choisi dans le groupe comprenant un produit concentré de la culture, un produit sec de la culture, des cellules obtenues par centrifugation de la culture, un produit sec des cellules, un produit lyophilisé des cellules, un produit des cellules traité par un agent tensioactif, un produit des cellules traité aux ultrasons, un produit des cellules disloqué mécaniquement, un produit des cellules traité au solvant, un produit des cellules traité enzymatiquement, une fraction protéique des cellules, un produit des cellules immobilisé et une préparation d'enzyme obtenue par extraction à partir des cellules.

3. Procédé selon la revendication 1, le glucide complexe accepteur étant un glucide complexe comprenant un oligosaccharide présentant du galactose sur son extrémité terminale non réductrice.

4. Procédé selon la revendication 1, le glucide complexe accepteur étant un glucide complexe comprenant un oligosaccharide présentant de l'acide sialique sur son extrémité terminale non réductrice.

5. Procédé selon la revendication 3, l'oligosaccharide qui présente du galactose sur son extrémité terminale non réductrice étant un oligosaccharide choisi dans le groupe comprenant le lactose, le globotriose, le *N*-acétyllactosamine, le lacto-*N*-tétraose, le lacto-*N-*néotétraose, le Lewis^{a} et le Lewis^{x}.

6. Procédé selon la revendication 4, l'oligosaccharide qui présente de l'acide sialique sur son extrémité terminale non réductrice étant un oligosaccharide choisi dans le groupe comprenant le NeuAcα2-3Galβ1-4Glc et le NeuAcα2-3Galβ1-4GlcNAc.

7. Procédé selon la revendication 1, le microorganisme étant un microorganisme appartenant au genre *Pasteurella.*

8. Procédé selon la revendication 7, le microorganisme appartenant au genre *Pasteurella* étant *Pasteurella multocida.*

9. Procédé selon la revendication 1, l'organisme génétiquement modifié étant un organisme génétiquement modifié qui comprend un ADN recombiné comportant un ADN codant ladite protéine présentant une activité α2,3/α2,8-sialyltransférase.

10. Procédé selon la revendication 9, l'organisme génétiquement modifié étant obtenu à l'aide d'un microorganisme comme cellule hôte.

11. Procédé selon la revendication 10, le microorganisme étant *Escherichia coli.*

12. Procédé selon la revendication 9, l'ADN codant la protéine présentant une activité α2,3/α2,8-sialyltransférase étant
(a) un ADN comprenant la séquence de nucléotides présentée en tant que Séquence n°2 ;
(b) ou un ADN qui s'hybride à un ADN constitué d'une séquence complémentaire à la séquence de nucléotides présentée en tant que Séquence n° 2 en conditions stringentes, et code une protéine présentant une activité α2,3/α2,8-sialyltransférase.


### SEQUENCE LISTING

<110> KYOWA HAKKO KOGYO CO., LTD.
<120> Process for producing alpha 2,3/alpha 2,8-sialyltransferase and complex carbohydrates containing sialic acid
<130> 11418WO1
<140>
   <141>
<150> JP 2001-292796
   <151> 2001-09-26
<160> 12
<170> PatentIn Ver. 2.0
<210> 1
   <211> 412
   <212> PRT
   <213> Pasteurella multocida
<400> 1
   Met Lys Asn Arg Arg Leu Asn Phe Lys Leu Phe Phe Leu Ile Ile Phe
     1               5                  10                  15
   Ser Leu Phe Ser Thr Leu Ser Trp Ser Lys Thr Ile Thr Leu Tyr Leu
                20                  25                  30
   Asp Pro Ala Ser Leu Pro Ala Leu Asn Gln Leu Met Asp Phe Thr Gln
            35                  40                  45
   Asn Asn Glu Asp Lys Thr His Pro Arg Ile Phe Gly Leu Ser Arg Phe
        50                  55                  60
   Lys Ile Pro Asp Asn Ile Ile Thr Gln Tyr Gln Asn Ile His Phe Val
   65 70 75 80
   Glu Leu Lys Asp Asn Arg Pro Thr Glu Ala Leu Phe Thr Ile Leu Asp
   85 90 95
   Gln Tyr Pro Gly Asn Ile Glu Leu Asp Ile His Leu Asn Ile Ala His
   100 105 110
   Ser Val Gln Leu Ile Arg Pro Ile Leu Ala Tyr Arg Phe Lys His Leu
   115 120 125
   Asp Arg Val Ser Ile Gln Arg Leu Asn Leu Tyr Asp Asp Gly Ser Met
   130 135 140
   Glu Tyr Val Asp Leu Glu Lys Glu Glu Asn Lys Asp Ile Ser Ala Glu
   145 150 155 160
   Ile Lys Gln Ala Glu Lys Gln Leu Ser His Tyr Leu Leu Thr Gly Lys
   165 170 175
   Ile Lys Phe Asp Asn Pro Thr Ile Ala Arg Tyr Val Trp Gln Ser Ala
   180 185 190
   Phe Pro Val Lys Tyr His Phe Leu Ser Thr Asp Tyr Phe Glu Lys Ala
   195 200 205
   Glu Phe Leu Gln Pro Leu Lys Glu Tyr Leu Ala Glu Asn Tyr Gln Lys
   210 215 220
   Met Asp Trp Thr Ala Tyr Gln Gln Leu Thr Pro Glu Gln Gln Ala Phe
   225 230 235 240
   Tyr Leu Thr Leu Val Gly Phe Asn Asp Glu Val Lys Gln Ser Leu Glu
   245 250 255
   Val Gln Gln Ala Lys Phe Ile Phe Thr Gly Thr Thr Thr Trp Glu Gly
   260 265 270
   Asn Thr Asp Val Arg Glu Tyr Tyr Ala Gln Gln Gln Leu Asn Leu Leu
   275 280 285
   Asn His Phe Thr Gln Ala Gly Gly Asp Leu Phe Ile Gly Asp His Tyr
   290 295 300
   Lys Ile Tyr Phe Lys Gly His Pro Arg Gly Gly Glu Ile Asn Asp Tyr
   305 310 315 320
   Ile Leu Asn Asn Ala Lys Asn Ile Thr Asn Ile Pro Ala Asn Ile Ser
   325 330 335
   Phe Glu Val Leu Met Met Thr Gly Leu Leu Pro Asp Lys Val Gly Gly
   340 345 350
   Val Ala Ser Ser Leu Tyr Phe Ser Leu Pro Lys Glu Lys Ile Ser His
   355 360 365
   Ile Ile Phe Thr Ser Asn Lys Gln Val Lys Ser Lys Glu Asp Ala Leu
   370 375 380
   Asn Asn Pro Tyr Val Lys Val Met Arg Arg Leu Gly Ile Ile Asp Glu
   385 390 395 400
   Ser Gln Val Ile Phe Trp Asp Ser Leu Lys Gln Leu
   405 410 412
<210> 2
   <211> 1236
   <212> DNA
   <213> Pasteurella multocida
<400> 2
   atg aaa aat cgt cga ctc aat ttc aaa ctg ttt ttc ctc atc att ttt 48
   Met Lys Asn Arg Arg Leu Asn Phe Lys Leu Phe Phe Leu Ile Ile Phe
   1 5 10 15
   tca tta ttc agc aca ctg agt tgg tca aaa aca atc acg ctg tat tta 96
   Ser Leu Phe Ser Thr Leu Ser Trp Ser Lys Thr Ile Thr Leu Tyr Leu
   20 25 30
   gat cct gcc tcc tta ccg gca tta aat cag ctg atg gac ttt acg caa 144
   Asp Pro Ala Ser Leu Pro Ala Leu Asn Gln Leu Met Asp Phe Thr Gln
   35 40 45
   aat aat gaa gat aaa aca cat cca cgt att ttt ggt ctt tct cgc ttt 192
   Asn Asn Glu Asp Lys Thr His Pro Arg Ile Phe Gly Leu Ser Arg Phe
   50 55 60
   aaa atc cct gac aac att att aca cag tat caa aat atc cat ttc gtc 240
   Lys Ile Pro Asp Asn Ile Ile Thr Gln Tyr Gln Asn Ile His Phe Val
   65 70 75 80
   gaa ctc aaa gat aat cgt ccc act gaa gca ctt ttt acg att ttg gat 288
   Glu Leu Lys Asp Asn Arg Pro Thr Glu Ala Leu Phe Thr Ile Leu Asp
   85 90 95
   caa tac cct ggt aac att gag tta gat ata cac tta aat att gct cat 336
   Gln Tyr Pro Gly Asn Ile Glu Leu Asp Ile His Leu Asn Ile Ala His
   100 105 110
   tcc gtt caa tta att cgt ccg att ttg gca tat cgt ttt aaa cat tta 384
   Ser Val Gln Leu Ile Arg Pro Ile Leu Ala Tyr Arg Phe Lys His Leu
   115 120 125
   gat cgt gta tca att cag cga tta aat ctt tat gac gat ggc tca atg 432
   Asp Arg Val Ser Ile Gln Arg Leu Asn Leu Tyr Asp Asp Gly Ser Met
   130 135 140
   gaa tat gtt gat tta gaa aaa gaa gaa aat aaa gat att tcc gca gaa 480
   Glu Tyr Val Asp Leu Glu Lys Glu Glu Asn Lys Asp Ile Ser Ala Glu
   145 150 155 160
   att aag caa gca gaa aaa caa ctt tct cac tat ttg ctt acc ggt aaa 528
   Ile Lys Gln Ala Glu Lys Gln Leu Ser His Tyr Leu Leu Thr Gly Lys
   165 170 175
   ata aaa ttt gat aac cca act att gct cgt tat gtc tgg caa tcc gcg 576
   Ile Lys Phe Asp Asn Pro Thr Ile Ala Arg Tyr Val Trp Gln Ser Ala
   180 185 190
   ttc cca gta aaa tat cat ttt tta agt aca gac tat ttt gaa aaa gcc 624
   Phe Pro Val Lys Tyr His Phe Leu Ser Thr Asp Tyr Phe Glu Lys Ala
   195 200 205
   gaa ttt tta caa cca cta aaa gaa tat tta gcg gaa aat tat caa aaa 672
   Glu Phe Leu Gln Pro Leu Lys Glu Tyr Leu Ala Glu Asn Tyr Gln Lys
   210 215 220
   atg gac tgg acc gct tac caa cag ctg act cca gaa cag caa gca ttc 720
   Met Asp Trp Thr Ala Tyr Gln Gln Leu Thr Pro Glu Gln Gln Ala Phe
   225 230 235 240
   tac tta aca ttg gta ggc ttc aat gac gaa gtc aag cag tcg cta gaa 768
   Tyr Leu Thr Leu Val Gly Phe Asn Asp Glu Val Lys Gln Ser Leu Glu
   245 250 255
   gtg caa caa gcc aaa ttt atc ttt acc ggc acg aca act tgg gaa gga 816
   Val Gln Gln Ala Lys Phe Ile Phe Thr Gly Thr Thr Thr Trp Glu Gly
   260 265 270
   aat acc gat gtg cga gaa tac tac gca cag caa caa ctt aat tta ctt 864
   Asn Thr Asp Val Arg Glu Tyr Tyr Ala Gln Gln Gln Leu Asn Leu Leu
   275 280 285
   aat cac ttt acc caa gct ggg ggc gat tta ttt att ggt gat cat tat 912
   Asn His Phe Thr Gln Ala Gly Gly Asp Leu Phe Ile Gly Asp His Tyr
   290 295 300
   aaa atc tac ttt aaa ggg cat cct aga ggt ggt gaa att aat gac tac 960
   Lys Ile Tyr Phe Lys Gly His Pro Arg Gly Gly Glu Ile Asn Asp Tyr
   305 310 315 320
   att ctg aac aat gct aaa aat atc acc aat atc cct gcc aat att tcc 1008
   Ile Leu Asn Asn Ala Lys Asn Ile Thr Asn Ile Pro Ala Asn Ile Ser
   325 330 335
   ttt gaa gta ttg atg atg aca ggc tta tta cct gat aaa gtg ggt ggt 1056
   Phe Glu Val Leu Met Met Thr Gly Leu Leu Pro Asp Lys Val Gly Gly
   340 345 350
   gtt gca agt tca ctg tat ttc tcc tta cca aaa gaa aaa att agc cat 1104
   Val Ala Ser Ser Leu Tyr Phe Ser Leu Pro Lys Glu Lys Ile Ser His
   355 360 365
   att att ttc aca tcg aat aaa caa gtg aaa agc aaa gaa gat gcg cta 1152
   Ile Ile Phe Thr Ser Asn Lys Gln Val Lys Ser Lys Glu Asp Ala Leu
   370 375 380
   aat aat ccg tat gtt aag gtc atg cgt cgt tta ggt ata att gac gaa 1200
   Asn Asn Pro Tyr Val Lys Val Met Arg Arg Leu Gly Ile Ile Asp Glu
   385 390 395 400
   tca caa gtc atc ttt tgg gac agt tta aaa cag ttg 1236
   Ser Gln Val Ile Phe Trp Asp Ser Leu Lys Gln Leu
   405 410
<210> 3
   <211> 308
   <212> PRT
   <213> Pasteurella multocida
<400> 3
   Met Asn Leu Ile Ile Cys Cys Thr Pro Leu Gln Val Leu Ile Ala Glu
   1 5 10 15
   Lys Ile Ile Ala Lys Phe Pro His Met Pro Phe Tyr Gly Val Met Leu
   20 25 30
   Ser Thr Val Ser Asn Lys Lys Phe Asp Phe Tyr Ala Lys Arg Leu Ala
   35 40 45
   Gln Gln Cys Gln Gly Phe Phe Ser Met Val Gln His Lys Asp Arg Phe
   50 55 60
   Asn Leu Leu Lys Glu Ile Leu Tyr Leu Lys Arg Thr Phe Ser Gly Lys
   65 70 75 80
   His Phe Asp Gln Val Phe Val Ala Asn Ile Asn Asp Leu Gln Ile Gln
   85 90 95
   Phe Leu Leu Ser Ala Ile Asp Phe Asn Leu Leu Asn Thr Phe Asp Asp
   100 105 110
   Gly Thr Ile Asn Ile Val Pro Asn Ser Leu Phe Tyr Gln Asp Asp Pro
   115 120 125
   Ala Thr Leu Gln Arg Lys Leu Ile Asn Val Leu Leu Gly Asn Lys Tyr
   130 135 140
   Ser Ile Gln Ser Leu Arg Ala Leu Ser His Thr His Tyr Thr Ile Tyr
   145 150 155 160
   Lys Gly Phe Lys Asn Ile Ile Glu Arg Val Glu Pro Ile Glu Leu Val
   165 170 175
   Ala Ala Asp Asn Ser Glu Lys Val Thr Ser Ala Val Ile Asn Val Leu
   180 185 190
   Leu Gly Gln Pro Val Phe Ala Glu Asp Glu Arg Asn Ile Ala Leu Ala
   195 200 205
   Glu Arg Val Ile Lys Gln Phe Asn Ile His Tyr Tyr Leu Pro His Pro
   210 215 220
   Arg Glu Lys Tyr Arg Leu Ala Gln Val Asn Tyr Ile Asp Thr Glu Leu
   225 230 235 240
   Ile Phe Glu Asp Tyr Ile Leu Gln Gln Cys Gln Thr His Lys Tyr Cys
   245 250 255
   Val Tyr Thr Tyr Phe Ser Ser Ala Ile Ile Asn Ile Met Asn Lys Ser
   260 265 270
   Asp Asn Ile Glu Val Val Ala Leu Lys Ile Asp Thr Glu Asn Pro Ala
   275 280 285
   Tyr Asp Ala Cys Tyr Asp Leu Phe Asp Glu Leu Gly Val Asn Val Ile
   290 295 300
   Asp Ile Arg Glu
   305 308
<210> 4
   <211> 924
   <212> DNA
   <213> Pasteurella multocida
<400> 4
   atg aat ttg att att tgt tgt aca ccg tta cag gtg ttg att gca gaa 48
   Met Asn Leu Ile Ile Cys Cys Thr Pro Leu Gln Val Leu Ile Ala Glu
   1 5 10 15
   aaa att atc gct aaa ttt ccg cat atg cca ttt tat ggc gtc atg ctt 96
   Lys Ile Ile Ala Lys Phe Pro His Met Pro Phe Tyr Gly Val Met Leu
   20 25 30
   tca aca gtc agt aat aaa aaa ttt gat ttt tat gca aag cgg ctt gcg    144
   Ser Thr Val Ser Asn Lys Lys Phe Asp Phe Tyr Ala Lys Arg Leu Ala
           35                  40                  45
   caa cag tgc caa ggt ttt ttt tcc atg gtg cag cat aag gat cgc ttc    192
   Gln Gln Cys Gln Gly Phe Phe Ser Met Val Gln His Lys Asp Arg Phe
       50                  55                  60
   aat cta tta aaa gaa att ctg tat tta aaa cga aca ttt tcg ggt aag    240
   Asn Leu Leu Lys Glu Ile Leu Tyr Leu Lys Arg Thr Phe Ser Gly Lys
   65                  70                  75                  80
   cac ttt gat cag gtt ttt gtg gca aac att aat gac tta caa att cag    288
   His Phe Asp Gln Val Phe Val Ala Asn Ile Asn Asp Leu Gln Ile Gln
                   85                  90                  95
   ttt tta tta agt gcc atc gac ttt aat ctg tta aat acc ttt gat gac    336
   Phe Leu Leu Ser Ala Ile Asp Phe Asn Leu Leu Asn Thr Phe Asp Asp
               100                 105                 110
   ggc aca att aat att gta ccg aat agt ctt ttt tac caa gat gac cct    384
   Gly Thr Ile Asn Ile Val Pro Asn Ser Leu Phe Tyr Gln Asp Asp Pro
           115                 120                 125
   gcc acg ttg cag cga aaa ctg att aat gtg ctg tta ggt aat aaa tac    432
   Ala Thr Leu Gln Arg Lys Leu Ile Asn Val Leu Leu Gly Asn Lys Tyr
       130                 135                 140
   agt att caa tca tta cgc gct tta tcc cat aca cac tac act att tat    480
   Ser Ile Gln Ser Leu Arg Ala Leu Ser His Thr His Tyr Thr Ile Tyr
   145                 150                 155                 160
   aaa ggc ttc aag aat att att gaa cgg gta gag ccg att gaa ttg gtc    528
   Lys Gly Phe Lys Asn Ile Ile Glu Arg Val Glu Pro Ile Glu Leu Val
                   165                 170                 175
   gca gca gat aac agt gaa aaa gtc act tca gcg gtg att aac gta ttg    576
   Ala Ala Asp Asn Ser Glu Lys Val Thr Ser Ala Val Ile Asn Val Leu
   180 185 190
   ctt ggg caa ccc gtt ttt gct gaa gat gaa cgc aat att gcc tta gcg 624
   Leu Gly Gln Pro Val Phe Ala Glu Asp Glu Arg Asn Ile Ala Leu Ala
   195 200 205
   gaa cgc gtg atc aaa caa ttt aat att cat tat tat ttg cct cat cca 672
   Glu Arg Val Ile Lys Gln Phe Asn Ile His Tyr Tyr Leu Pro His Pro
   210 215 220
   cgc gaa aag tat cgt tta gcc caa gtc aat tac att gat acg gaa ttg 720
   Arg Glu Lys Tyr Arg Leu Ala Gln Val Asn Tyr Ile Asp Thr Glu Leu
   225 230 235 240
   atc ttt gaa gat tat att ctt cag caa tgt caa acc cac aaa tac tgt 768
   Ile Phe Glu Asp Tyr Ile Leu Gln Gln Cys Gln Thr His Lys Tyr Cys
   245 250 255
   gtt tat aca tat ttt agt agc gcc att att aat atc atg aat aaa agt 816
   Val Tyr Thr Tyr Phe Ser Ser Ala Ile Ile Asn Ile Met Asn Lys Ser
   260 265 270
   gac aat att gaa gtg gta gca tta aaa att gac aca gaa aat ccc gcc 864
   Asp Asn Ile Glu Val Val Ala Leu Lys Ile Asp Thr Glu Asn Pro Ala
   275 280 285
   tat gat gct tgt tat gat ttg ttt gat gag cta ggc gtt aac gtt att 912
   Tyr Asp Ala Cys Tyr Asp Leu Phe Asp Glu Leu Gly Val Asn Val Ile
   290 295 300
   gat ata aga gag 924
   Asp Ile Arg Glu
   305
<210> 5
   <211> 303
   <212> PRT
   <213> Pasteurella multocida
<400> 5
   Met Asp Lys Phe Ala Glu His Glu Ile Pro Lys Ala Val Ile Val Ala
   1 5 10 15
   Gly Asn Gly Glu Ser Leu Ser Gln Ile Asp Tyr Arg Leu Leu Pro Lys
   20 25 30
   Asn Tyr Asp Val Phe Arg Cys Asn Gln Phe Tyr Phe Glu Glu Arg Tyr
   35 40 45
   Phe Leu Gly Asn Lys Ile Lys Ala Val Phe Phe Thr Pro Gly Val Phe
   50 55 60
   Leu Glu Gln Tyr Tyr Thr Leu Tyr His Leu Lys Arg Asn Asn Glu Tyr
   65 70 75 80
   Phe Val Asp Asn Val Ile Leu Ser Ser Phe Asn His Pro Thr Val Asp
   85 90 95
   Leu Glu Lys Ser Gln Lys Ile Gln Ala Leu Phe Ile Asp Val Ile Asn
   100 105 110
   Gly Tyr Glu Lys Tyr Leu Ser Lys Leu Thr Ala Phe Asp Val Tyr Leu
   115 120 125
   Arg Tyr Lys Glu Leu Tyr Glu Asn Gln Arg Ile Thr Ser Gly Val Tyr
   130 135 140
   Met Cys Ala Val Ala Ile Ala Met Gly Tyr Thr Asp Ile Tyr Leu Thr
   145 150 155 160
   Gly Ile Asp Phe Tyr Gln Ala Ser Glu Glu Asn Tyr Ala Phe Asp Asn
   165 170 175
   Lys Lys Pro Asn Ile Ile Arg Leu Leu Pro Asp Phe Arg Lys Glu Lys
   180 185 190
   Thr Leu Phe Ser Tyr His Ser Lys Asp Ile Asp Leu Glu Ala Leu Ser
   195 200 205
   Phe Leu Gln Gln His Tyr His Val Asn Phe Tyr Ser Ile Ser Pro Met
   210 215 220
   Ser Pro Leu Ser Lys His Phe Pro Ile Pro Thr Val Glu Asp Asp Cys
   225 230 235 240
   Glu Thr Thr Phe Val Ala Pro Leu Lys Glu Asn Tyr Ile Asn Asp Ile
   245 250 255
   Leu Leu Pro Pro His Phe Val Tyr Glu Lys Leu Gly Thr Ile Val Ser
   260 265 270
   Lys Lys Ser Arg Phe His Ser Asn Leu Ile Val Arg Leu Ile Arg Asp
   275 280 285
   Leu Leu Lys Leu Pro Ser Ala Leu Lys His Tyr Leu Lys Glu Lys
   290 295 300 303
<210> 6
   <211> 909
   <212> DNA
   <213> Pasteurella multocida
<400> 6
   atg gat aag ttc gca gaa cat gaa ata ccg aaa gcc gtt att gtt gca 48
   Met Asp Lys Phe Ala Glu His Glu Ile Pro Lys Ala Val Ile Val Ala
   1 5 10 15
   ggg aat ggc gag agt tta agt caa att gat tat agg ttg tta ccg aaa 96
   Gly Asn Gly Glu Ser Leu Ser Gln Ile Asp Tyr Arg Leu Leu Pro Lys
   20 25 30
   aat tat gat gtg ttt cgt tgt aat caa ttt tat ttt gaa gaa cgc tat   144
   Asn Tyr Asp Val Phe Arg Cys Asn Gln Phe Tyr Phe Glu Glu Arg Tyr
           35                  40                  45
   ttt tta gga aac aag ata aaa gca gtt ttc ttc acg cca ggg gtc ttt   192
   Phe Leu Gly Asn Lys Ile Lys Ala Val Phe Phe Thr Pro Gly Val Phe
       50                  55                  60
   ctt gag caa tat tat aca ctt tat cat ctc aag aga aac aat gag tat   240
   Leu Glu Gln Tyr Tyr Thr Leu Tyr His Leu Lys Arg Asn Asn Glu Tyr
   65                  70                  75                  80
   ttt gtt gat aat gtg att ctc tct tct ttt aat cat cct aca gta gat   288
   Phe Val Asp Asn Val Ile Leu Ser Ser Phe Asn His Pro Thr Val Asp
                   85                  90                  95
   tta gaa aag agt cag aaa ata caa gca ctt ttt att gat gtg atc aac   336
   Leu Glu Lys Ser Gln Lys Ile Gln Ala Leu Phe Ile Asp Val Ile Asn
               100                 105                 110
   gga tat gaa aag tat tta tct aaa ctc act gct ttt gat gtt tat ttg   384
   Gly Tyr Glu Lys Tyr Leu Ser Lys Leu Thr Ala Phe Asp Val Tyr Leu
           115                 120                 125
   cgc tat aaa gaa tta tat gag aat caa aga att aca tct ggc gta tat   432
   Arg Tyr Lys Glu Leu Tyr Glu Asn Gln Arg Ile Thr Ser Gly Val Tyr
       130                 135                 140
   atg tgt gca gtt gct att gcg atg gga tat aca gat att tac tta act   480
   Met Cys Ala Val Ala Ile Ala Met Gly Tyr Thr Asp Ile Tyr Leu Thr
   145                 150                 155                 160
   ggt atc gat ttt tat caa gcg agc gaa gaa aac tac gca ttc gat aat   528
   Gly Ile Asp Phe Tyr Gln Ala Ser Glu Glu Asn Tyr Ala Phe Asp Asn
                   165                 170                 175
   aaa aag cct aac att att agg tta ttg cct gat ttt cgg aaa gaa aaa 576
   Lys Lys Pro Asn Ile Ile Arg Leu Leu Pro Asp Phe Arg Lys Glu Lys
   180 185 190
   aca ctc ttt tct tat cat agt aaa gat att gat ttg gaa gca tta tct 624
   Thr Leu Phe Ser Tyr His Ser Lys Asp Ile Asp Leu Glu Ala Leu Ser
   195 200 205
   ttt tta caa cag cat tat cat gtt aat ttt tat tca att tca cca atg 672
   Phe Leu Gln Gln His Tyr His Val Asn Phe Tyr Ser Ile Ser Pro Met
   210 215 220
   agc cct ttg tct aaa cat ttt cct att cca act gta gag gat gat tgt 720
   Ser Pro Leu Ser Lys His Phe Pro Ile Pro Thr Val Glu Asp Asp Cys
   225 230 235 240
   gaa aca act ttt gtt gcg cca cta aaa gaa aat tac att aat gat ata 768
   Glu Thr Thr Phe Val Ala Pro Leu Lys Glu Asn Tyr Ile Asn Asp Ile
   245 250 255
   ttg ttg cct cct cat ttt gta tat gaa aaa tta ggg acc atc gtg tct 816
   Leu Leu Pro Pro His Phe Val Tyr Glu Lys Leu Gly Thr Ile Val Ser
   260 265 270
   aag aaa tca cgt ttt cat tct aac ttg att gtc agg ttg att aga gac 864
   Lys Lys Ser Arg Phe His Ser Asn Leu Ile Val Arg Leu Ile Arg Asp
   275 280 285
   tta ttg aaa tta ccg agt gca ctt aaa cac tat tta aaa gaa aaa 909
   Leu Leu Lys Leu Pro Ser Ala Leu Lys His Tyr Leu Lys Glu Lys
   290 295 300
<210> 7
   <211> 29
   <212> DNA
   <213> Artificial Sequence
<220>
   <223> Description of Artificial Sequence:Synthetic DNA
<400> 7
   atcatcgata tgaaaaatcg tcgactcaa 29
<210> 8
   <211> 29
   <212> DNA
   <213> Artificial Sequence
<220>
   <223> Description of Artificial Sequence:Synthetic DNA
<400> 8
   tatggatcct tacaactgtt ttaaactgt 29
<210> 9
   <211> 29
   <212> DNA
   <213> Artificial Sequence
<220>
   <223> Description of Artificial Sequence:Synthetic DNA
<400> 9
   gtaatcgatg acggctaaaa gtagg 25
<210> 10
   <211> 32
   <212> DNA
   <213> Artificial Sequence
<220>
   <223> Description of Artificial Sequence:Synthetic DNA
<400> 10
   ttcggatcct tactctctta tatcaataac gt 32
<210> 11
   <211> 29
   <212> DNA
   <213> Artificial Sequence
<220>
   <223> Description of Artificial Sequence:Synthetic DNA
<400> 11
   ggaatcgata tggataagtt cgcagaaca 29
<210> 12
   <211> 29
   <212> DNA
   <213> Artificial Sequence
<220>
   <223> Description of Artificial Sequence:Synthetic DNA
<400> 12
   taaggatcct tatttttctt ttaaatagt 29